(19) Europäisches Patentamt / European Patent Office / Office européen des brevets

(11) **EP 3 927 366 B1**

(12) **EUROPEAN PATENT SPECIFICATION**

(45) Date of publication and mention
of the grant of the patent:
**25.09.2024 Bulletin 2024/39**

(21) Application number: **20759283.3**

(22) Date of filing: **19.02.2020**

(51) International Patent Classification (IPC):
*A61K 9/51* (2006.01)      *A61K 38/17* (2006.01)
*A61K 38/45* (2006.01)      *A61K 38/46* (2006.01)
*A61K 45/06* (2006.01)      *A61K 38/16* (2006.01)
*A61K 47/58* (2017.01)      *A61K 47/62* (2017.01)
*A61K 47/69* (2017.01)      *A61K 39/00* (2006.01)
*A61P 25/00* (2006.01)      *A61P 35/04* (2006.01)
*C07K 16/28* (2006.01)

(52) Cooperative Patent Classification (CPC):
**A61P 35/04; A61K 9/0019; A61K 9/5138;**
**A61K 38/164; A61K 38/1767; A61K 45/06;**
**A61K 47/58; A61K 47/62; A61K 47/6925;**
**A61P 25/00;** A61K 9/5153; A61K 2039/505;
A61K 2039/55555; C07K 16/2887; C07K 2319/74

(86) International application number:
**PCT/US2020/018811**

(87) International publication number:
**WO 2020/172263 (27.08.2020 Gazette 2020/35)**

(54) **DELIVERY OF MACROMOLECULES INTO THE CENTRAL NERVOUS SYSTEM VIA THE BLOODSTREAM**

VERABREICHUNG VON MAKROMOLEKÜLEN IN DAS ZENTRALNERVENSYSTEM ÜBER DIE BLUTBAHN

APPORT DE MACROMOLÉCULES AU SYSTÈME NERVEUX CENTRAL PAR L'INTERMÉDIAIRE DE LA CIRCULATION SANGUINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2019 US 201962807370 P**

(43) Date of publication of application:
**29.12.2021 Bulletin 2021/52**

(73) Proprietor: **THE REGENTS OF THE UNIVERSITY OF CALIFORNIA**
Oakland, CA 94607-5200 (US)

(72) Inventors:
• **KAMATA, Masakazu**
Los Angeles, California 90024 (US)
• **CHEN, Irvin S.Y.**
Palos Verdes Estates, California 90274 (US)
• **LU, Yunfeng**
Culver City, California 90230 (US)
• **WEN, Jing**
Culver City, California 90230 (US)

(74) Representative: **Murgitroyd & Company**
**165-169 Scotland Street**
**Glasgow G5 8PL (GB)**

(56) References cited:
EP-A1- 3 424 498      WO-A2-2008/054544
WO-A2-2010/104865    CN-A- 105 012 952
US-A1- 2018 256 511   US-A1- 2018 369 159

- JIA XIANGQIAN ET AL: "In situpolymerization on biomacromolecules for nanomedicines", NANO RESEARCH, TSINGHUA UNIVERSITY PRESS, CN, vol. 11, no. 10, 16 May 2018 (2018-05-16), pages 5028 - 5048, XP036607468, ISSN: 1998-0124, [retrieved on 20180516], DOI: 10.1007/S12274-018-2080-2
- GAN LIU ET AL: "Phosphorylcholine-based stealthy nanocapsules enabling tumor microenvironment-responsive doxorubicin release for tumor suppression", THERANOSTICS, vol. 7, no. 5, 1 January 2017 (2017-01-01), AU, pages 1192 - 1203, XP055628146, ISSN: 1838-7640, DOI: 10.7150/thno.17881
- ZHU ET AL.: "Enzyme-Responsive Delivery of Multiple Proteins with Spatiotemporal Control", ADVANCED MATERIALS, vol. 27, 12 May 2015 (2015-05-12), pages 3620 - 3625, XP055733093
- WEBSTER ET AL.: "Brain penetration, target engagement, and disposition of the blood-brain barrier-crossing bispecific antibody antagonist of metabotropic glutamate receptor type 1", THE FASEB JOURNAL, vol. 30, no. 5, 2 February 2016 (2016-02-02), pages 1927 - 1940, XP055733095
- JIANG ET AL.: "CXCL13 drives spinal astrocyte activation and neuropathic pain via CXCR5", JCI, vol. 126, no. 2, 11 January 2016 (2016-01-11), pages 745 - 761, XP055733096

**Description**

TECHNICAL FIELD

The invention relates to protein nanocapsules capable of delivering therapeutic agents to the central nervous system.

BACKGROUND OF THE INVENTION

Treatment regimens which deliver therapeutic agents such as polypeptides to diseased tissues *in vivo* can provide ultimate therapy options for many refractory pathologies including certain metastatic cancers. However, the use of peptide drugs and therapeutic proteins is limited by their poor stability and/or permeability in certain physiological environments. Thus, there is a growing effort to circumvent these problems by designing nanostructures that can act as carriers for the delivery of therapeutic proteins such antibodies.

Treatments for cancer metastases, especially those of the central nervous system (CNS), are less successful than those for primary tumors (1). Approximately 15%-40% of all cancers develop a CNS metastasis (2,3), which most commonly arises from lung cancer, melanoma, breast cancer, and colorectal cancer. Therapeutic monoclonal antibodies (mAbs) have revolutionized the treatment of cancer; however, their efficacy is limited in patients with CNS metastases due to insufficient mAb CNS delivery-typically 0.1% of the levels in plasma (4). By bypassing the blood-brain barrier (BBB) through intrathecal or intraventricular administration, mAb therapy has shown some effectiveness against CNS tumor metastases (4-10). However, direct CNS administration is invasive, with potential for neurotoxicity, and is limited by rapid efflux of antibodies from the CNS within hours (5,10,11). Therefore, novel approaches for mAbs delivery are preferable to maintain systemic therapeutic effect in the CNS with improved efficiency.

To date, various carrier vehicles for macromolecule delivery such as viral vectors, liposomes, cationic polymers, inorganic delivery systems, and other biomolecules have been explored to improve CNS delivery (12-14). Viral vectors are effective for CNS delivery in some settings but have potential safety concerns (15,16). Liposome-based protein delivery has been shown to penetrate the BBB, but with relatively low efficiency, biocompatibility and stability (17,18). Polymer nanoparticles conjugated to target ligands with a variety of structures and morphologies have been used to form micelles through self-assembly, but in vivo instability, tissue-specific accumulations and protein denaturation during complexing are problematic (19,20). Inorganic delivery systems, including gold nanoparticles (21,22) and mesoporous silica particles (23), are non-biodegradable and difficult to load or conjugate with macromolecules. Biomolecules, such as cell-penetrating peptides and antibodies, have improved the efficacy of macromolecule delivery, but degradation of cargo still hampers their therapeutic applications (24). The above approaches have shown promise, but drastic improvements are needed-especially in the systemic delivery of macromolecules into the CNS (19,25). Patent document EP3424498 discloses nanocapsules having a brain tumor targeting delivery effect.

Rituximab (RTX) for treatment of non-Hodgkin lymphoma (NHL) was the first anticancer antibody approved by the U.S. Food and Drug Administration. RTX binds to CD20+ lymphoma cells and induces cell death through complement-dependent cytotoxicity (CDC), antibody-dependent cell-mediated cytotoxicity (ADCC), and apoptosis (26). RTX may also promote anti-lymphoma immune responses (27). The substantial benefits of RTX administration in treatments for systemic NHL are well-established, but treatment of primary and relapsed CNS lymphoma has not been effective through the intravenous route, likely due to the very low levels of systemic RTX entering the CNS (4). CNS involvement in NHL is relatively rare, but there is an elevated risk in patients with immunodeficiency diseases[9] or renal, cardiac, lung, and liver transplantation.

Central nervous system (CNS) metastases are a major cause of cancer deaths with unfortunately few therapeutic options. While monoclonal antibody-based therapy for cancer is a powerful therapeutic strategy, such therapies are typically limited in CNS metastases due to insufficient antibody delivery to the CNS. Consequently, there is a need in this field of technology for methods and materials capable of delivering therapeutic agents such as antibodies to the central nervous system.

SUMMARY OF THE INVENTION

It will be appreciated that the scope is in accordance with the claims. In a general context, the present invention relates to nano-encapsulated therapeutic compositions designed to include selected constellations of elements that, for example, allows them to be administrated intravenously and then cross the blood brain barrier into the central nervous system. The present invention further provides methods of making and using these nano-encapsulated therapeutic compositions. The compositions and methods disclosed herein allow medical practitioners to more easily deliver therapeutic agents such as cancer specific antibodies into the central nervous system, and in this way overcome significant problems in this field of technology. Accordingly, there is provided nanoencapsulated therapeutic composition as defined in claim 1, a method of making said composition as defined in claim 2 and said composition for use in delivering a therapeutic agent to the central nervous system as defined in claim 5. Further features are provided in accordance with the dependent claims. The specification may include description of arrangements outside the scope of the claims provided as background and to assist in understanding the invention.In working embodiments of the invention presented herein, we demonstrate that, as compared to administration of native antibody Rituximab (RTX), timed-release nanocapsule delivery of RTX achieves levels around 10-fold higher RTX concentration in the CNS following a single-course treatment and is maintained for at least 4 weeks, as opposed to 1 week with native RTX. Furthermore, we developed a human NHL xenograft murine model for CNS metastases and show therapeutic efficacy of RTX nanocapsules against CNS lymphomas. In addition, using a humanized BLT mouse model of human brain cancer, we demonstrate clearance of the CNS lymphomas.

The invention disclosed herein has a number of embodiments as defined in the claims. Embodiments of the invention include a composition of matter as defined in claim 1 comprising: a polypeptide cargo antibody; a polymeric network configured to form a polymer shells using, for example, neutral monomers with zwitterionic properties, and 2-methacryloyloxyethyl phosphorylcholine to form a nanocapsule that encapsulates the polypeptide cargo antibody; and hydrolysable crosslinking moieties comprising a glycerol dimethacrylate and/or a Poly(DL-lactide)-b-Poly(ethylene glycol)-b-Poly(DL-lactide)-diacrylate triblock copolymer coupled to polymers forming the polymeric network of the nanocapsule and a targeting agent CXCL13 coupled to the polymeric network, selected for its ability to preferentially bind to targets present in tissues of the central nervous system.. In such embodiments of the invention, the nanocapsule is formed *in situ* on the polypeptide cargo; the polymeric network and the hydrolysable crosslinking moiety and their relative amounts are disposed in a three-dimensional architecture so that: the nanocapsule crosses blood brain barriers to deliver nanocapsules in a bloodstream into a central nervous system; and the hydrolysable crosslinking moiety is cleaved in the central nervous system so as to release the polypeptide cargo from the nanocapsule. Optionally such embodiments include a plurality of nanocapsules, wherein constituents and ratios of the nanocapsule components in the plurality of nanocapsules are disposed in a three-dimensional architecture so that the plurality of nanocapsules release the polypeptide cargos at different rates of time. In certain embodiments of the invention, the constituents and ratios of the shell components, the polypeptide cargo, the polymeric network and the hydrolysable crosslinking moiety are disposed in a three-dimensional architecture so that about 10-fold more protein cargo crosses blood brain barriers as compared with the protein cargo antibody in the absence of the nanocapsule.

Specifically, claim 1 is directed to a composition of matter comprising an antibody; a polymeric network formed from 2-methacryloyloxyethyl phosphorylcholine and configured to form a nanocapsule that encapsulates the antibody; a hydrolysable crosslinking moiety comprising a glycerol dimethacrylate and/or a Poly(DL-lactide)-b-Poly(ethylene glycol)-b-Poly(DL-lactide)-diacrylate triblock copolymer coupled to polymers forming the polymeric network; and a targeting agent comprising CXCL13 coupled to the polymeric network. In this embodiment of the invention, the nanocapsule is formed *in situ* on the polypeptide cargo; and the nanocapsule constituents and ratios of the polypeptide cargo, the polymeric network, the hydrolysable crosslinking moiety and the targeting agent are disposed in a three-dimensional architecture so that the nanocapsule crosses blood brain barriers to deliver nanocapsules in a bloodstream into a central nervous system such that at least 2, 5 or 10 fold more antibody that is disposed in the nanocapsule crosses blood brain barriers as compared with the antibody in the absence of the nanocapsule; and the hydrolysable crosslinking moiety is cleaved in the central nervous system so as to release the antibody from the nanocapsule.

Another embodiment of the invention is a method of making a polypeptide cargo antibody (e.g. an antibody selected to target cancerous cells) encapsulated by a polymeric network as defined in claim 2. Typically, the method comprises performing an *in situ* polymerization process on monomers comprising a 2-methacryloyloxyethyl phosphorylcholine moiety) that localize to the surface of the polypeptide cargo via molecular affinity interactions (e.g. electrostatic interactions and the like) so as to form a polymerized nanocapsule that encapsulates the polypeptide cargo; coupling polymers in the polymeric network with one or more hydrolysable crosslinking moieties, wherein the hydrolysable crosslinking moieties are selected to cleaved in the central nervous system so as to release the polypeptide cargo from the nanocapsule; and then coupling the polymeric network to one or more targeting agents CXCL13 (e.g. a polypeptide comprising a CXCL13 motif), wherein the targeting agents are selected to bind to targets present in tissues of the central nervous system. In such embodiments, the nanocapsule constituents and ratios of the polypeptide cargo antibody, the polymeric network, the hydrolysable crosslinking moiety and the targeting agent are selected to allow the nanocapsule to cross blood brain barriers to deliver protein cargo antibodies into a central nervous system; and also the hydrolysable crosslinking moiety to be cleaved in the central nervous system so as to release the polypeptide cargo antibody from the nanocapsule.

Yet another embodiment of the invention is a method of selectively delivering a therapeutic agent to the central nervous system of an individual, the method comprising intravenously administering a nanocapsule composition defined in claim 5 to the individual; and then allowing the composition to cross the blood brain barrier of the individual into the central nervous system. In this context, the composition comprises an antibody, a polymeric network formed from 2-methacryloyloxyethyl phosphorylcholine and configured to form a nanocapsule that encapsulates the antibody, a hydrolysable crosslinking moiety coupled to polymers forming the polymeric network, and a targeting agent CXCL13 (e.g. one that targets diseased tissue within the central nervous system) coupled to the polymeric network. In such embodiments of the invention, the nanocapsule is formed *in situ* on the polypeptide cargo, the nanocapsule constituents and ratios of the antibody, the polymeric network, the hydrolysable crosslinking moiety and the targeting agent are disposed in a three-dimensional architecture so that the nanocapsule crosses blood brain barriers to deliver nanocapsules present in a bloodstream into a central nervous system such that at least 2 fold more antibody disposed in nanocapsules crosses blood brain barriers as compared with the antibody not encapsulated by nanocapsules. Typically in these embodiments, the hydrolysable crosslinking moiety is cleaved in the central nervous system so as to release the antibody from the nanocapsule.

Other objects, features and advantages of the present invention will become apparent to those skilled in the art from the following detailed description. It is to be understood, however, that the detailed description and specific examples, while indicating some embodiments of the present invention, are given by way of illustration and not limitation.

BRIEF DESCRIPTION OF THE DRAWINGS

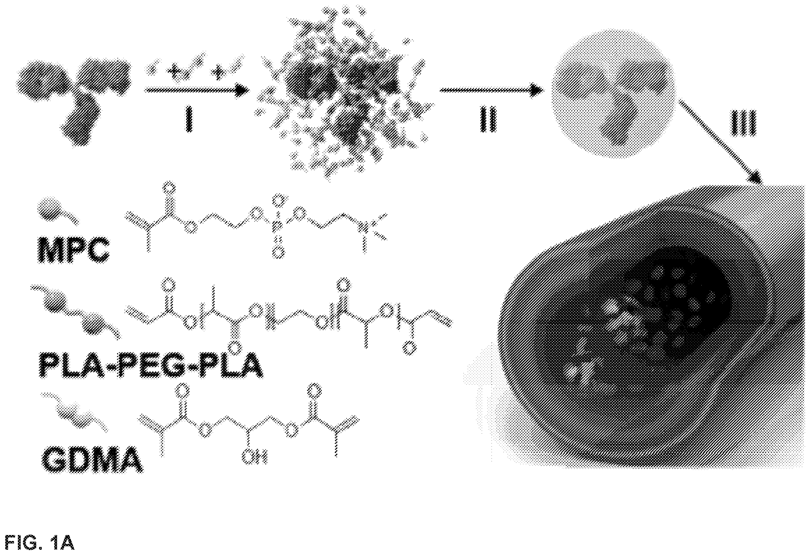

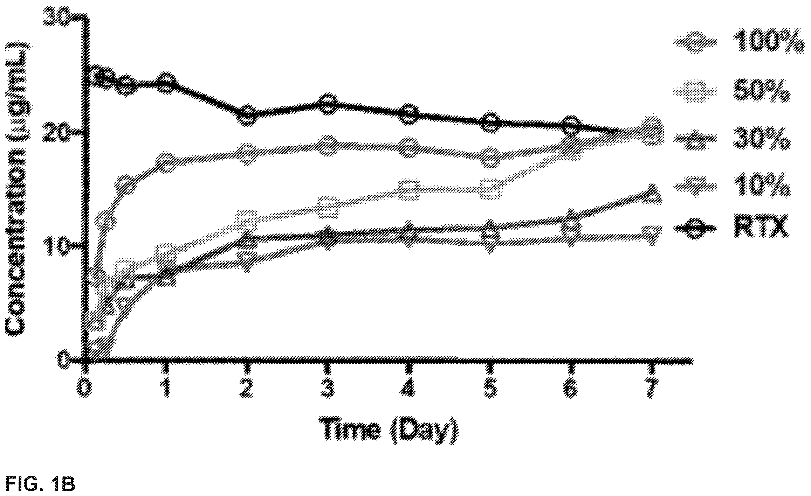

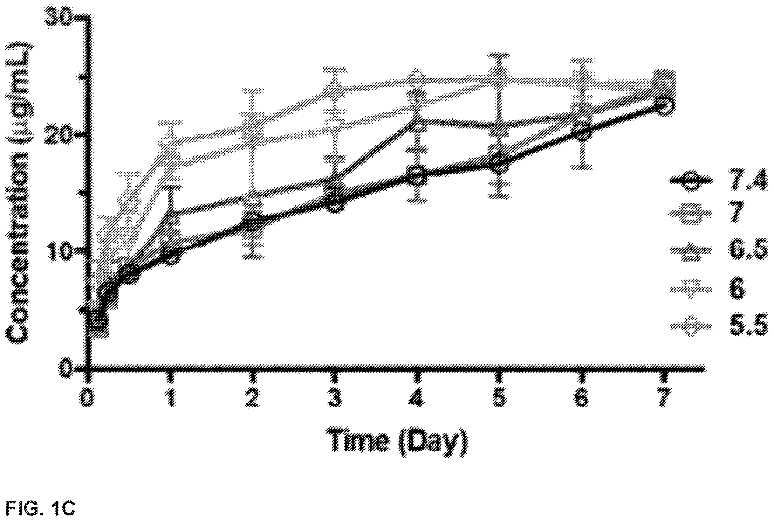

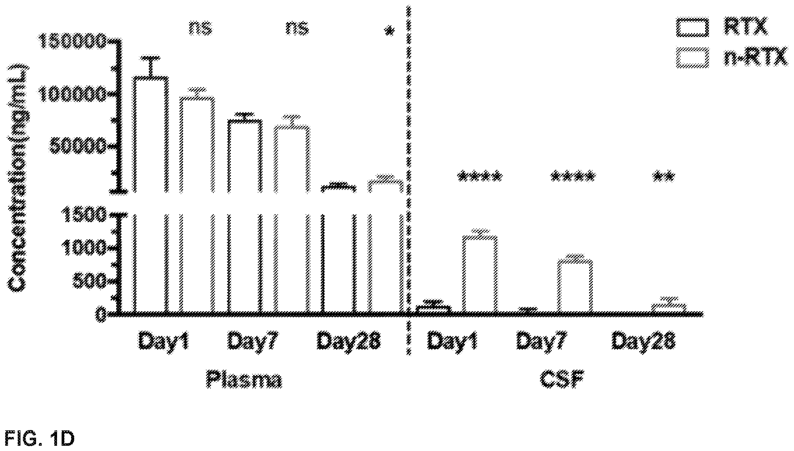

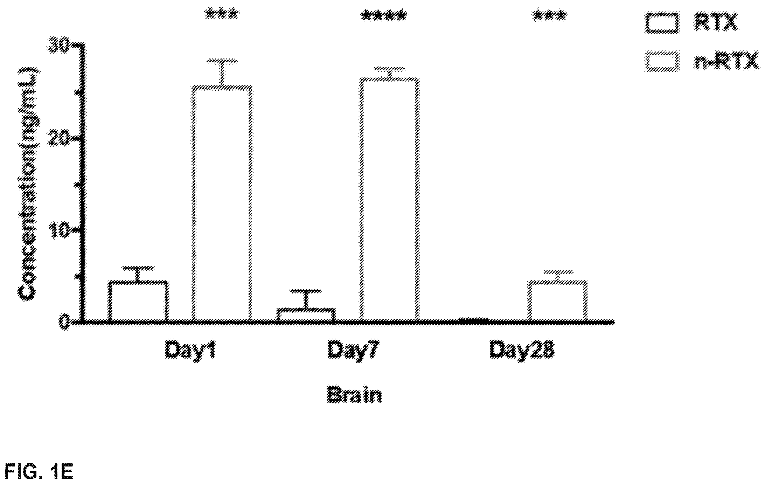

**Figures 1A-1E provide data showing that timed-release nanocapsules facilitate brain delivery of RTX in mice.** Figure 1(A): Scheme of the synthesis and release of timed-release RTX nanocapsules by (I) enriching MPC monomer and hydrolysable crosslinker, PLA-PEG-PLA, and slowly hydrolysable crosslinker, GDMA, around a RTX molecule, (II) *in situ* polymerization of the monomer and crosslinkers forming a thin shell of polymer around a RTX molecule, and (III) releasing RTX when polymer shells are degraded under physiological condition by hydrolysis of the crosslinkers. Figure 1(B): RTX nanocapsules were synthesized with mixed crosslinkers GDMA and PLA-PEG-PLA, at which the ratios of PLA-PEG-PLA were 100%, 50%, 30%, and 10%. Twenty-five $\mu$g of each nanoencapsulated RTX or native RTX (RTX) was added into 1mL of PBS and incubated at 37 °C for 1 week. The concentration of free RTX were measured by ELISA. Figure 1(C): The release rates of RTX nanocapsules with 50% PLA-PEG-PLA crosslinkers were compared in PBS buffers with various pH values presenting physiological conditions (pH=7.4 and 7) and acidic pH conditions (pH=6.5, 6, and 5.5). Twenty-five $\mu$g of each samples was added into either 1mL of PBS buffer and incubated at 37 °C for up to 1 week. The concentration of free RTX were measured by ELISA. Figure 1(D) and Figure 1(E) B6 mice (n=3) were administrated with 20 mg/kg of native RTX or RTX nanocapsules with 50% PLA-PEG-PLA crosslinkers (n-RTX) via retro-orbital vein. Plasma, CSF, and perfused brain tissue samples were collected and concentrations of free RTX were measured by ELISA on days 1, 7, and 28. Data are shown means $\pm$ standard deviations (SDs) of triplicate measurements. The statistical significance was calculated to native RTX group using a p-value, *: P value< 0.0332; **: P values <0.0021; ***: P value<0.0002, ****: P value<0.0001. ns: not significant.

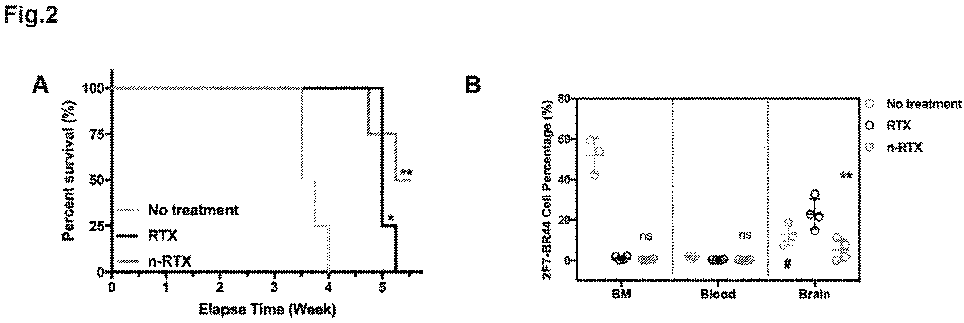

**Figures 2A-2B provide data showing that nanoencapsulation enhances anti-lymphoma activity of RTX against CNS lymphomas in the 2F7-BR44 murine xenograft model.** 2F7-BR44 cells ($2\times10^6$/animal) in 100 $\mu$L Hank's balanced salt solution were injected into NSG mice via tail vein. Five days after injection, mice were randomly separated into three groups: no treatment (n=3), native RTX and n-RTX treatment (n=4). Native RTX or n-RTX was injected via retro-orbital vein at 4 mg/kg/day for 5 days. Figure 2(A): Kaplan-Meier survival curves of the mice in each group were plotted relative to the number of weeks after 2F7-BR44 cell injection. Figure 2(B): Tissue distribution of 2F7-BR44 cells in blood, perfused bone marrow (BM) and brain was analyzed by flow cytometry at endpoints. Data are shown means $\pm$ standard deviations (SDs). The statistical significance was calculated to no treatment group using a p-value, *: P value< 0.0332; **: P values <0.0021; ns: not significant. #: Note that the apparent higher tumor burden in brain of the native RTX group compared to the no-treatment group is due to one-week longer duration of the native RTX group versus the no-treatment group which were euthanized one week earlier due to massive systemic tumor growth.

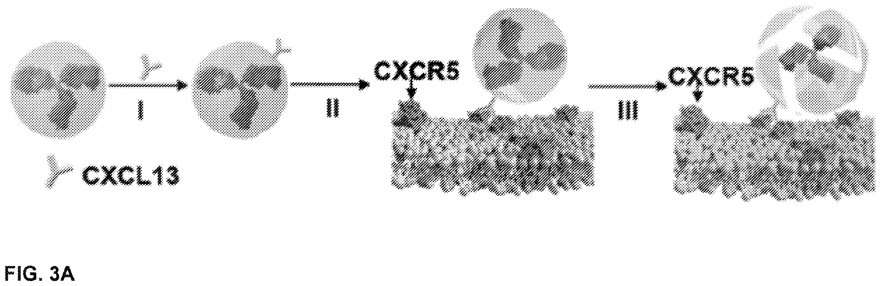

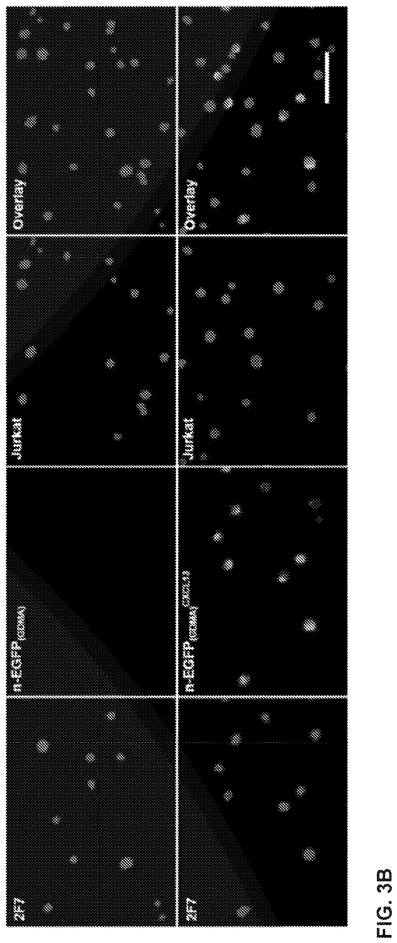

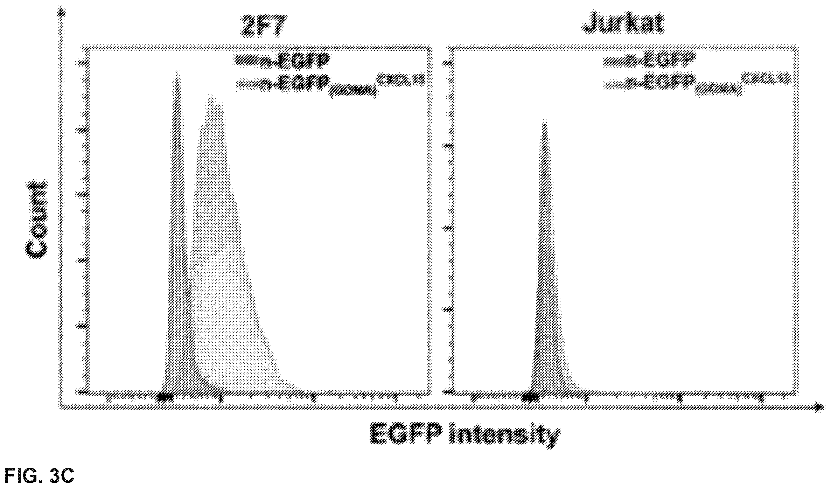

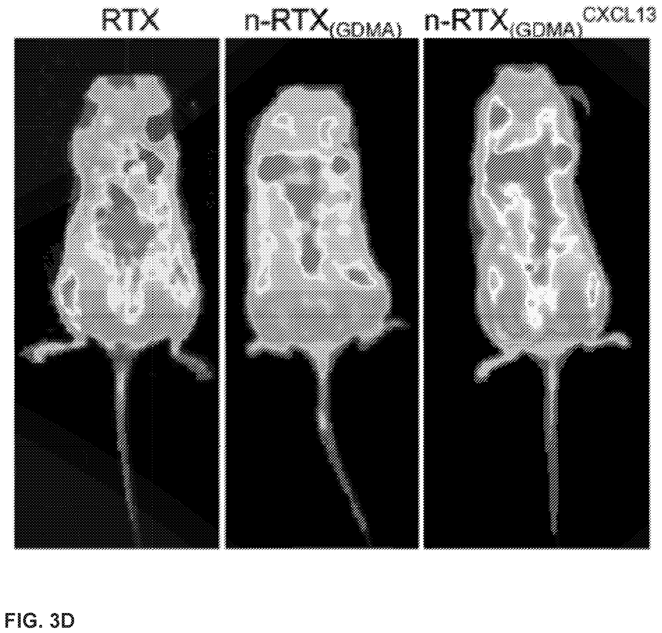

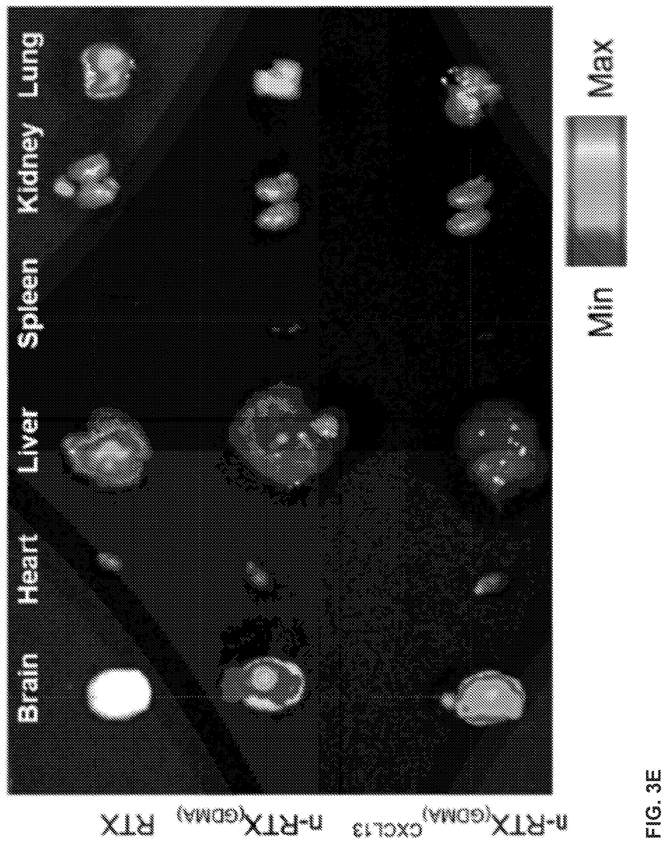

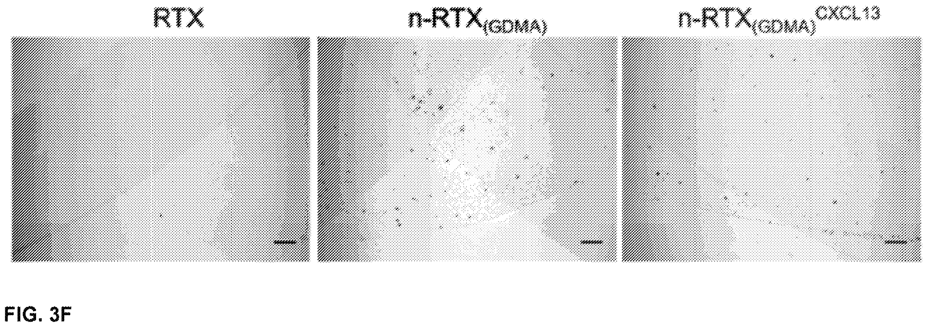

**Figures 3A-3F provide data showing that CXCL13 conjugation mediates 2F7-BR44 cell specific nanocapsule targeting without affecting overall biodistribution.** Figure 3(A): Scheme of CXCL13 conjugation on n-RTX$_{(GDMA)}$. CXCL13 was conjugated to n-RTX$_{(GDMA)}$ at a molar ratio of 1:1 through copper-free click chemistry. Figure 3(B) and Figure 3(C): EGFP nanocapsules with 100% slowly hydrolysable crosslinkers, GDMA, (n-EGFP$_{(GDMA)}$) conjugated with or without CXCL13 (0.1 mg/ml each) were incubated 1 hour at 37°C with a mixed cell population of 2F7-BR44 cells (2F7, mStrawberry+) and Jurkat T cells (Jurkat, BFP+). The binding of each nanocapsule on each cell population was analyzed on Leica DMi8 inverted microscope in Figure 3(B) or on BD LSRFortessa in Figure 3(C). Figure 3(D) and Figure 3(E): NSG mice were administrated with 20 mg/kg of carboxytetramethylrhodamine (TAMRA)-labelled RTX, n-RTX and n-RTX$^{CXCL13}$ via tail vein. Fluorescence images of whole body Figure 3(D) and each organ Figure 3(E) were taken 1 day after administration. Figure 3(F): NSG mice were administrated with 20 mg/kg of gold-nanoparticle labelled RTX, n-RTX$_{(GDMA)}$ and n-RTX$_{(GDMA)}^{CXCL13}$ via tail vein. Brain tissues were obtained 1 day after administration and processed for immunohistochemical analysis. Gold nanoparticles in paraffin sections were analyzed following silver enhancement, in which present as black depositions. Bar=50 μm.

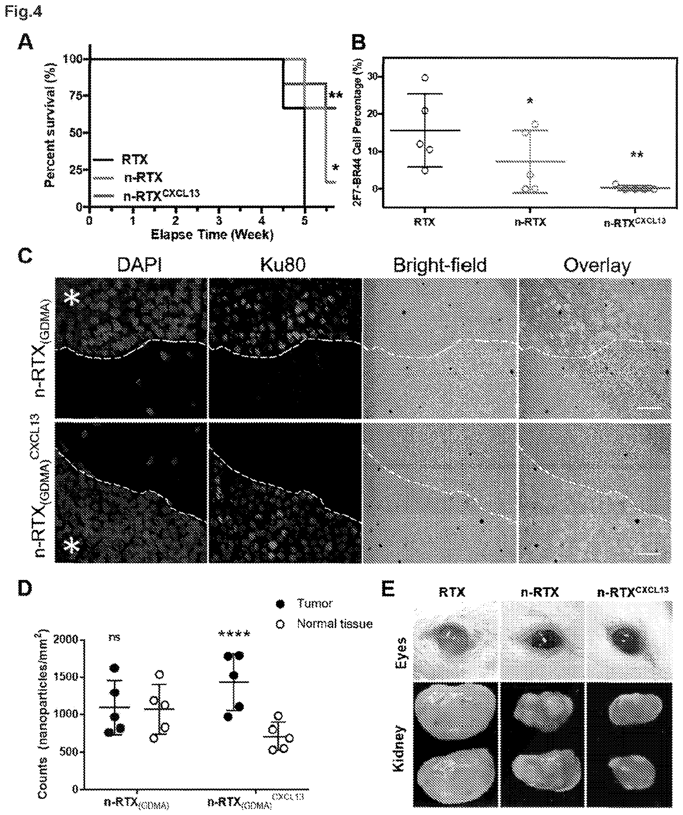

**Figures 4A-4E provide data showing that CXCL13 conjugation improves anti-lymphoma activity of n-RTX.** Figure 4(A) and Figure 4(B): 2F7-BR44 cells were injected to NSG mice via tail vein ($2\times10^6$/animal). Five days after the injection, mice were randomly separated into three groups (n=5): native RTX, n-RTX, and n-RTX$^{CXCL13}$. Each form of RTX was administrated via retro-orbital injection at 4mg/kg/day for 5 days. Kaplan-Meier survival curves of the mice in each group were plotted relative to the number of weeks after 2F7-BR44 cell injection in Figure 4(A). Percentages of 2F7-BR44 cells in the brain were analyzed by flow cytometry at endpoints following cardiac perfusion in Figure 4(B). The statistical significance was calculated to native RTX group using a p-value, *: P value<0.0332; **: P values <0.0021. Figure 4(C) and Figure 4(D): Localizations of gold-labelled n-RTX$_{(GDMA)}$ with or without CXCL13 conjugation were analyzed in the brain of 2F7-BR44 xenograft NSG mice cells 3 weeks after xenograft. One day after administration of gold-labelled n-RTX$_{(GDMA)}$ or n-RTX$_{(GDMA)}$$^{CXCL13}$ via retro-orbital injection, brain tissue samples were processed for immunohistochemical analysis. Gold nanoparticles in paraffin sections were analyzed following silver enhancement, in which present as black depositions. 2F7-BR44 cells were stained with anti-human Ku80 antibody and rhodamine red-X anti-rabbit IgG. The nuclei were stained by 4',6-diamidino-2-phenylindole (DAPI). Asterisks indicate the tumor site while the white dash curves illustrate the boundary of tumor-normal parenchyma. Bar=25 μm in Figure 4(C). The average counts of gold-labelled n-RTX$_{(GDMA)}$ or n-RTX$_{(GDMA)}$$^{CXCL13}$ per mm$^2$ from 5 spots (total counts from samples administrated with n-RTX$_{(GDMA)}$ or n-RTX$_{(GDMA)}$$^{CXCL13}$ are approximately 11,000, respectively) in both the tumor site and normal tissue site were quantified by ImageJ. The statistical significance was calculated to n-RTX$_{(GDMA)}$ using a p-value, *: P values <0.0332; ns: not significant as shown in Figure 4(D). Figure 4(E): Representative images of intraocular lymphoma and lymphoma surrounding the kidney in each group.

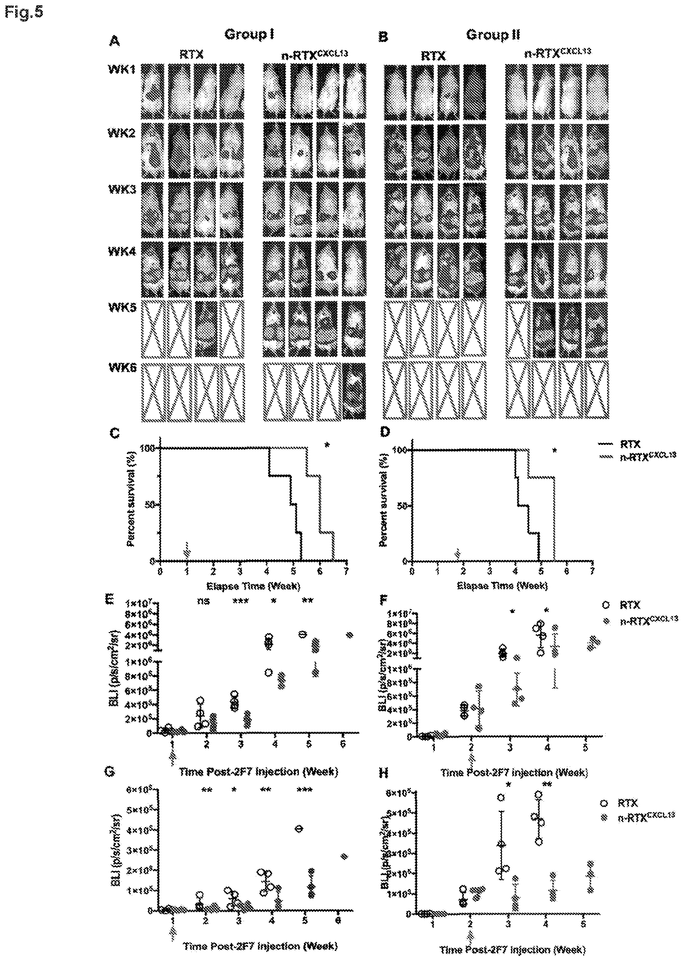

**Figs. 5A-5H provide data showing that CXCL13 conjugation improves anti-lymphoma activity of n-RTX at different stages of lymphoma progression.** CXCL13 was conjugated to n-RTX at a molar ratio of 1:1 through copper-free click chemistry (n-RTX$^{CXCL13}$). 2F7-BR44 cells expressing firefly luciferase (2F7-BR44-Luc) were injected to NSG mice via tail vein ($2\times10^6$/animal). Mice were randomly separated into four groups (n=4): native RTX or n-RTX$^{CXCL13}$ treatment at week 1 (Group I) or week 2 (Group II) after 2F7-BR44-Luc cell injection. Each form of RTX was injected via retro-orbital vein at 4 mg/kg/day for 5 days. The tumor progression and metastasis status were monitored weekly by using bioluminescence imaging on IVIS Lumina II In Vivo Imaging system. Red arrows show the treatment time in each figure. Figure 5(A) and Figure 5(B): Expansion and metastasis status of 2F7-BR44-Luc cells in NSG mice treated with native RTX or n-RTX$^{CXCL13}$ at week 1 (Group I) or week 2 (Group II) as visualized by bioluminescence imaging. The red X boxes represent deceased mice. Sensitivity settings were adjusted at each time point to maintain 500-5000 counts per pixel and assigned the same color scale for both groups. Figure 5(C) and Figure 5(D): Kaplan-Meier survival curves of the mice in each group were plotted relative to the number of weeks after 2F7-BR44-Luc cell injection. Figure 5(E) and Figure 5(F): The bioluminescence intensity (BLI) in the whole body was compared between mice with treatment of native RTX and n-RTX$^{CXCL13}$ in both groups and expressed in photons per second per square centimeter per steradian (p/s/cm$^2$/sr). Figure 5(G) and Figure 5(H): The BLI from head area was compared between mice with treatment of native RTX and n-RTX$^{CXCL13}$ in both groups. The statistical significance was calculated to native RTX group using a p-value, *: P value<0.0332; **: P values <0.0021. ***: P values <0.0002. ns: not significant.

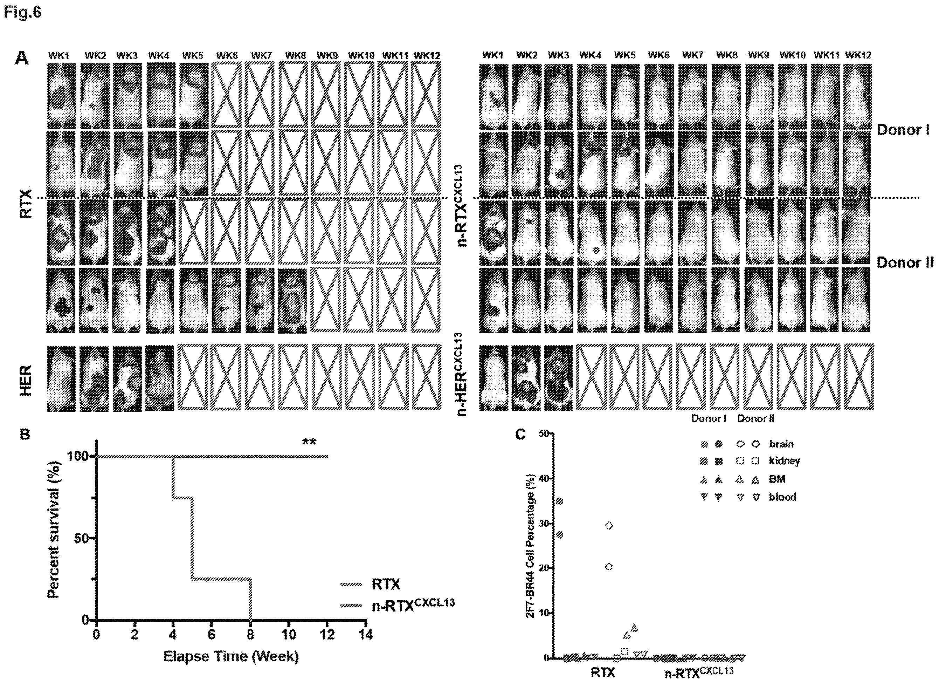

**Figures 6A-6C provide data showing that n-RTX$^{CXCL13}$ exhibits superior anti-lymphoma activity against CNS lymphomas in the 2F7-BR44-Luc cell xenograft humanized mice.** 2F7-BR44-Luc cells ($2\times10^6$/animal) were injected into humanized BLT mice generated from two independent donors (Donors I and II) via tail vein. Mice were randomly separated into two groups for treatment by native RTX or n-RTX$^{CXCL13}$ at week 1 after the injection. Each form of RTX was administrated via retro-orbital vein at 4 mg/kg/day for 5 days. The tumor progression status was tracked weekly using bioluminescence imaging on IVIS Lumina II In Vivo Imaging system. Figure 6(A): Biodistribution of 2F7-BR44-Luc cells in humanized mice treated with native RTX or n-RTX$^{CXCL13}$ at week 1 as visualized by luciferase bioimaging. The red X boxes represent deceased mice. Sensitivity settings were adjusted at each time point to maintain 250-5000 counts per pixel and assigned the same color scale for all treatment groups. Figure 6(B): Kaplan-Meier survival curves of the mice were plotted relative to the number of weeks after injection. Necropsies were performed at the end of the experiment (Week 13). Figure 6(C): Percentages of 2F7-BR44-Luc cells in tissues were assessed by flow cytometry following cardiac perfusion at the endpoints. HER: naive Herceptin. n-HER: Herceptin nanocapsule. The statistical significance was calculated to native RTX group using a p-value, **: P values <0.0021.

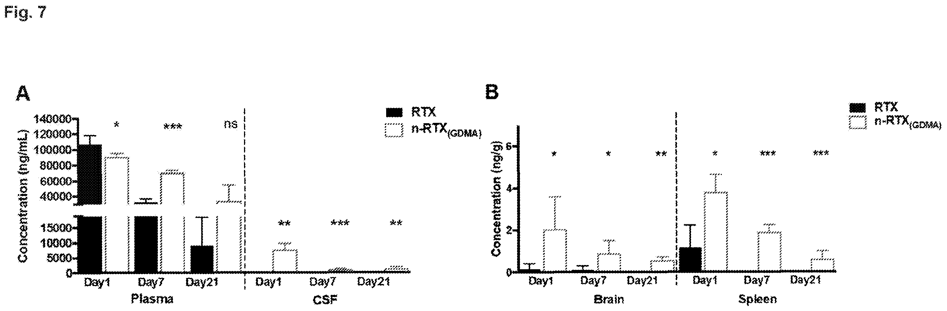

**Figures 7A and 7B provide data showing that Nanocapsules with slowly degradable crosslinker under physiological conditions, GDMA, (n-RTX(GDMA)) improve RTX delivery in central nervous system (CNS) in mice.** Native RTX or n-RTX(GDMA) was injected in B6 mice (n=3) via retro-orbital vein (20mg/kg). The concentration of native and encapsulated RTX in mouse body fluids (plasma and CSF in Figure (7A)) and perfused tissue homogenates (brain and spleen in Figure (7B)) was measured by ELISA at day 1, day 7 and day 21 after retro-orbital vein injection. To release RTX from n-RTX(GDMA), mouse body fluids and tissue homogenates were pretreated with 50 mM sodium acetate buffer (pH 5.4) at 4°C for overnight before ELISA measurement. Data are shown means ± standard deviations (SDs) of triplicate measurements. The statistical significance was calculated to native RTX group using a p-value, *: P value< 0.0332; **: P values <0.0021; ***: P value<0.0002. ns: not significant.

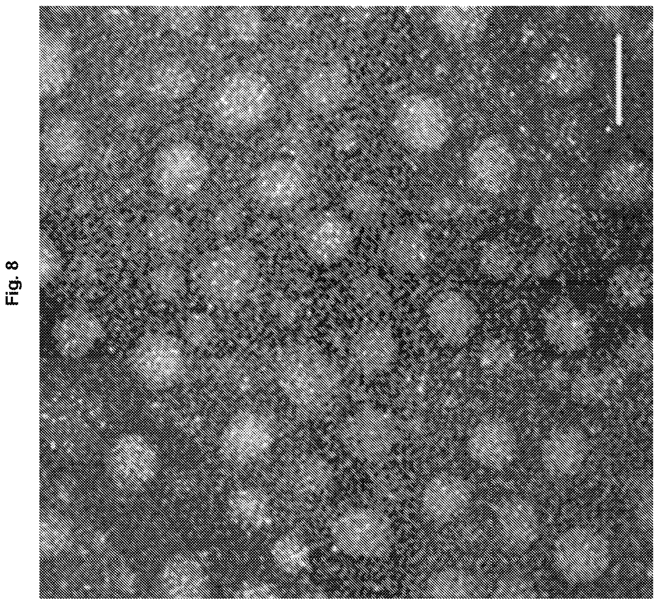

**Figure 8 provides data showing that RTX nanocapsules exhibit a spherical nanoscale architecture.** To monitor the morphology of nanocapsules, n-RTX were negatively stained by 2% uranyl acetate for 1-2 minutes at room temperature. TEM images were obtained using a Philips EM120 TEM. Bar = 50nm.

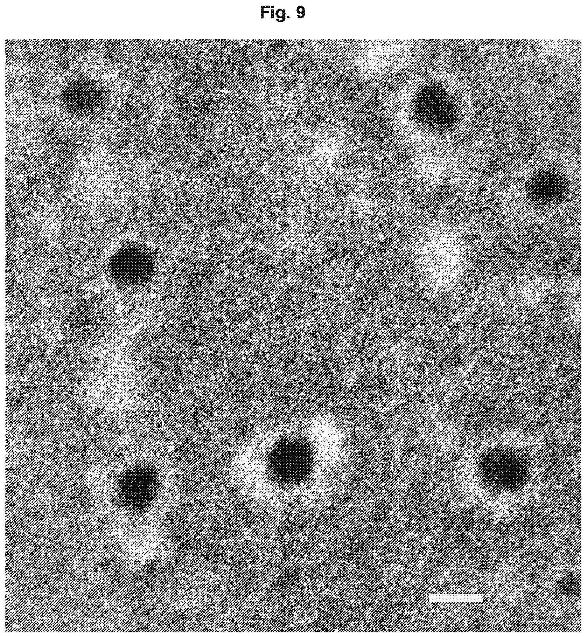

**Figure 9 provides data showing that RTX nanocapsules contain a single RTX molecule within a spherical nanoscale architecture.** For detection of RTX in each nanocapsule, RTX was labeled by a 1.4nm single nanogold particle using mono-sulfo-N-hydroxy-succinimido-nanogold prior encapsulation. Nanocapsules with gold-labeled RTX were mounted on the TEM grid and negatively stained by 2% uranyl acetate solution following silver enhancement by the GoldENhance EM kit for the gold nanoparticle. TEM images were obtained using a Philips EM120 TEM. Bar = 10nm.

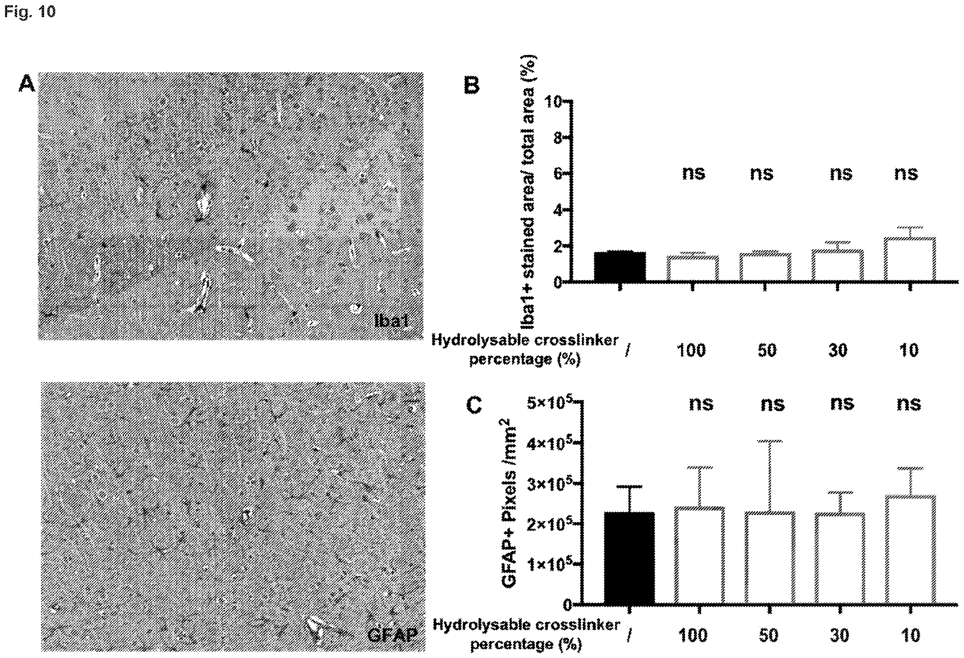

**Figures 10A-10C provide data showing that n-RTX treatment mediates no significant level of brain damage.** Sprague Dawley rats were injected with native RTX or n-RTX via tail vein at a dosage of 15mg/kg. Figure 10(A): IHC analysis of rat brain samples was performed on 4-5 sections in the parasagittal plane that included the hippocampal formation as well as granule and pyramidal cell layers. Sections were immunostained with primary antibody to either Iba1 (microglial marker) or GFAP (astrocyte marker). Micrographs are shown at 20x magnification. Representative images of immunohistochemical staining were shown. Figure 10(B) and Figure 10(C): A positive pixel count in rat brain treated with native RTX (black filled bars) and n-RTX (red open bars) was quantified using NIH ImageJ for Iba1 and an Aperio ImageScope algorithm for GFAP. Data are expressed means ± SD of triplicate slides. The statistical significance was calculated to native RTX group using a p-value, ns: not significant.

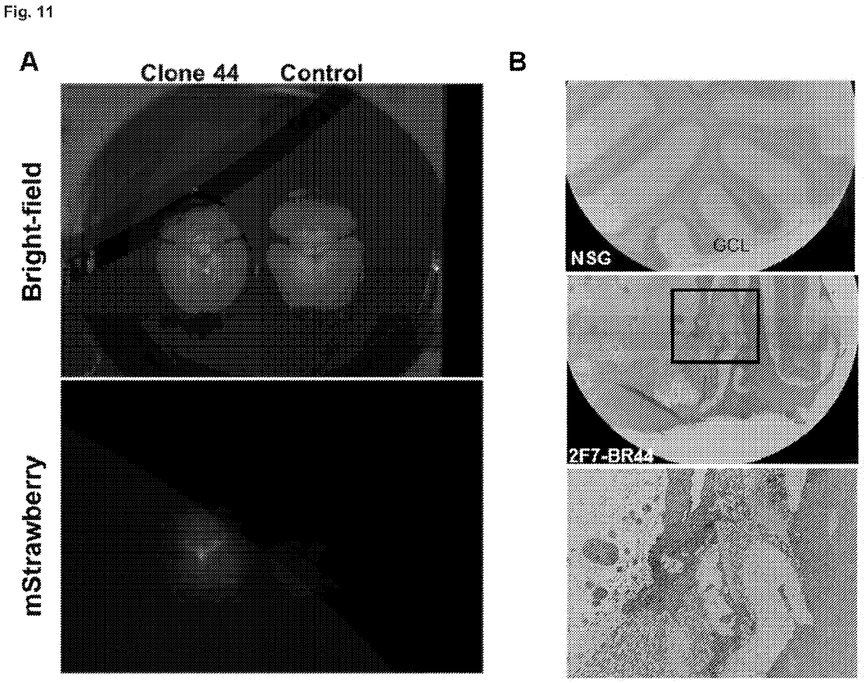

**Figures 11A and 11B provide data showing that 2F7-BR44 clone mediates CNS metastasis in a xenograft NSG mouse model.** Figure 11(A): Single-cell derived cloned 2F7 cells (clone #44) were injected into NSG mice via intraperitoneal injection (2x106/animal). Brain tissues were obtained at week 7 post-2F7-clone #44 cell injection and an mStrawberry fluorescent image was taken with Maestro In vivo Imaging system. Figure 11(B): 2F7-BR44 cells were injected to NSG mice via intraperitoneal injection (2×106/animal). Brain tissues were obtained at 4 weeks post-injection and processed for IHC analysis. The mid-sagittal sections of the cerebellum were served for Cresyl violet staining to detect Nissl bodies (blue). 2F7-BR44 cells were detected by anti-human CD20 antibody, followed by HRP-labeled secondary antibody (brown). Top panel, normal NSG brain. 4x magnification. Middle panel, brain tissue at week 4 following tail vein injection of 2F7-BR44 cells. 4x magnification. Bottom panel, 10x magnification of the boxed region from the middle panel. GCL: granule cell layer.

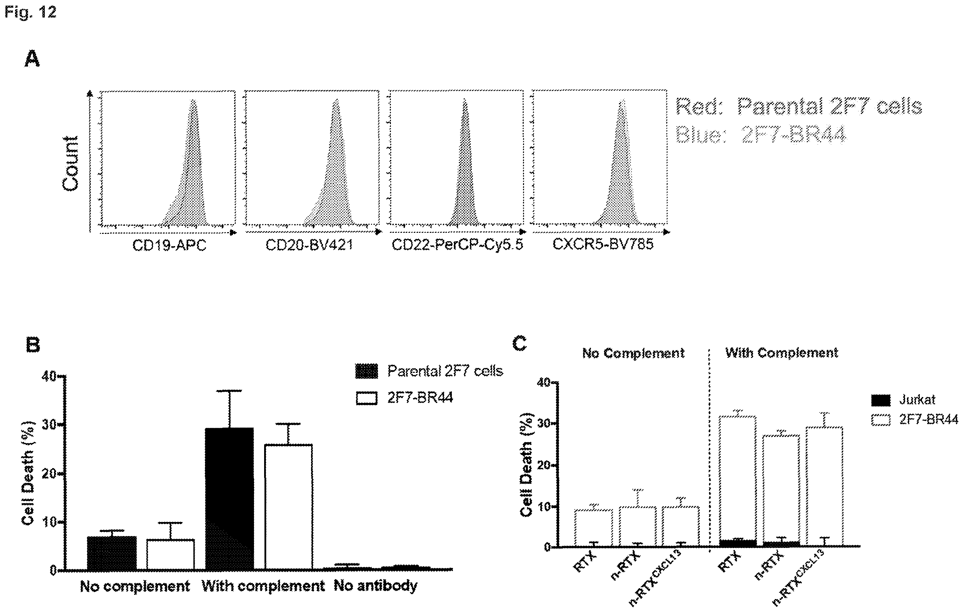

**Figures 12A-12C provide data showing that 2F7-BR44 clone shows similar levels of four B cell surface markers and RTX sensitivity compared to the parental 2F7 cells.** Figure 12(A): Parental 2F7 cells and 2F7-BR44 cells (2 × 106 each) were stained with four B cell surface markers- CD19, CD20, CD22, and CXCR5-and compared to investigate levels of surface expression by flow cytometry. Figure 12(B): RTX mediating cytotoxicity was assessed against parental 2F7 and 2F7-BR44 cells. Parental 2F7 and 2F7-BR44 cells were cultured in 24-well plates (1x106/mL) for 24 hours in the presence of RTX (20 μg/mL) and 20% non-heat inactivated human serum as a source of complements (With complement). Cells cultured with no human serum (No complement) or no RTX addition (No antibody) were used as negative controls. Same volumes of cell culture were taken, and absolute cell numbers of each condition were counted on MACSQuant® Analyzer 10. Figure 12(C): Cell-type specific cytotoxicity mediated by native RTX, n-RTX, and n-RTX$^{CXCL13}$ were compared in mixed 2F7-BR44 and Jurkat T cell culture with or without complements. Cell death percentages were calculated as cell death (%)= 100×(1-(cell number after rituximab treatment/control without rituximab)). Data are shown means ± SDs. The statistical significance was calculated to no antibody group using a p-value, *: P value< 0.0332; **: P values <0.0021; ***: P value<0.0002, ****: P value<0.0001. ns: not significant.

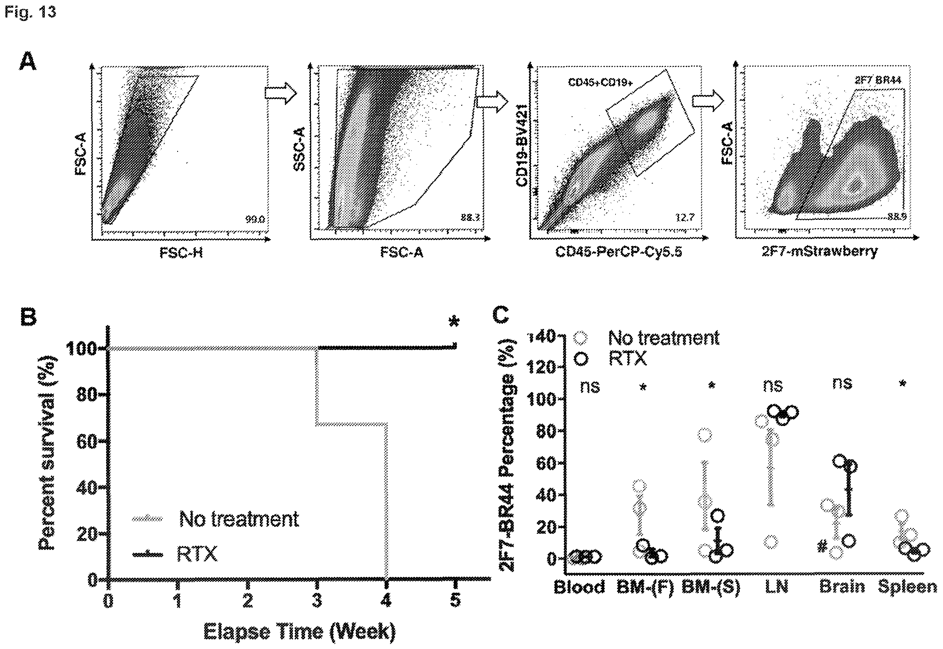

**Figures 13A-13C provide data showing that Anti-lymphoma efficacy of native RTX is limited in brains of the 2F7-BR44 lymphoma xenograft model.** 2F7-BR44 cells (2x106/animal) stably expressing mStrawberry as a marker were injected into NSG mice via tail vein (n=3 per group). Five days after lymphoma cell injection, mice were randomly separated into two groups: No treatment and native RTX treatment (RTX: 4mg/kg/day for 5 days). Figure 13(A): Cells isolated from blood, bone marrow, lymph nodes, brain, and spleen were stained with anti-human CD19 and CD45 antibodies, followed by analysis of single cell populations on BD LSRFortessa. 2F7-BR44 cells were gated as a double positive population for human CD45 and CD19, followed by gating for the mStrawberry+ population. Same gates were applied to all other samples. The 2F7-BR44 cell percentage was calculated as CD45+CD19+mStrawberry+ population against FSC-A/SSC-A gate. Flow panels shown are a representative of a brain sample. Figure 13(B): Kaplan-Meier survival curves of the mice in each group were plotted relative to the number of weeks after 2F7-BR44 cell injection. Figure 13(C): Tissue distribution of 2F7-BR44 cells were analyzed by flow cytometry at endpoints. #: Note that the apparent higher tumor burden in brain of the RTX group compared to the untreated group is due to one-week longer duration of the RTX group versus the untreated group which were euthanized one week earlier due to massive systemic tumor growth. The statistical significance was calculated to no treatment group using a p-value, *: P<0.05. BM-(F): bone marrow from femur; BM-(S): bone marrow from sternum; LNs: lymph nodes.

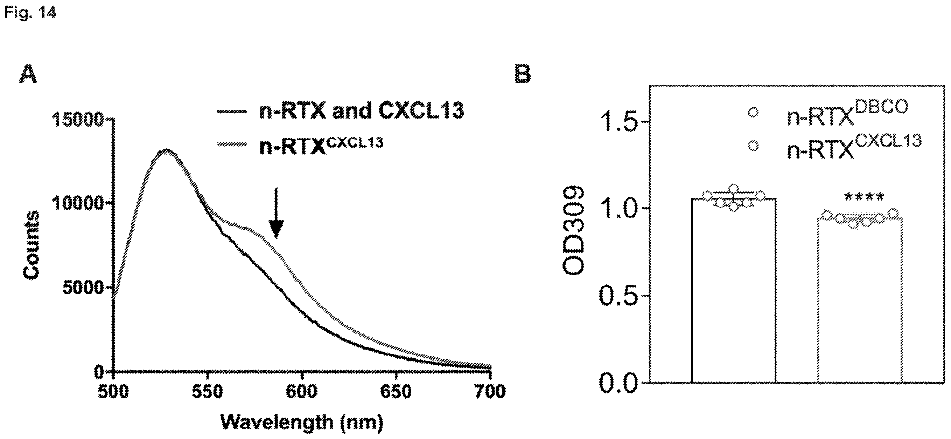

**Figures 14A-14B provide data showing that CXCL13 is conjugated to n-RTX at a molar ratio of approximately 1:1.** Figure 14(A): The fluorescence spectra of n-RTX labeled with FITC and CXCL13 labeled with tetramethylrhodamine (TRITC) were collected before (n-RTX and CXCL13, black line) and after (n-RTXCXCL13, red line) conjugation. Clear emission spectrum appeared around 580 nm, indicating that the binding between n-RTX and CXCL13 caused energy transfer. Figure 14(B): Quantification of CXCL13 numbers on each nanocapsule. The n-RTX was labeled with DBCO groups using dibenzocyclooctyne-S-S-N-hydroxysuccinimidyl ester (DBCO-SS-NHS), while CXCL13 was labeled with NHS-PEG4-azide. The number of DBCO-conjugated n-RTX was determined by the adsorption at 309 nm based on the Beer-Lambert law as described in the Methods. The average number of DBCO groups on the DBCO-modified n-RTX was measured as 8.8 per nanocapsule. The number was determined as 7.6 per nanocapsule after CXCL13 conjugation, indicating that the average number of CXCL13 in nanocapsules is 1.2 per nanocapsule. Error bars represent the mean $\pm$ standard error of the mean from 6 independent experiments, ****P<0.0001.

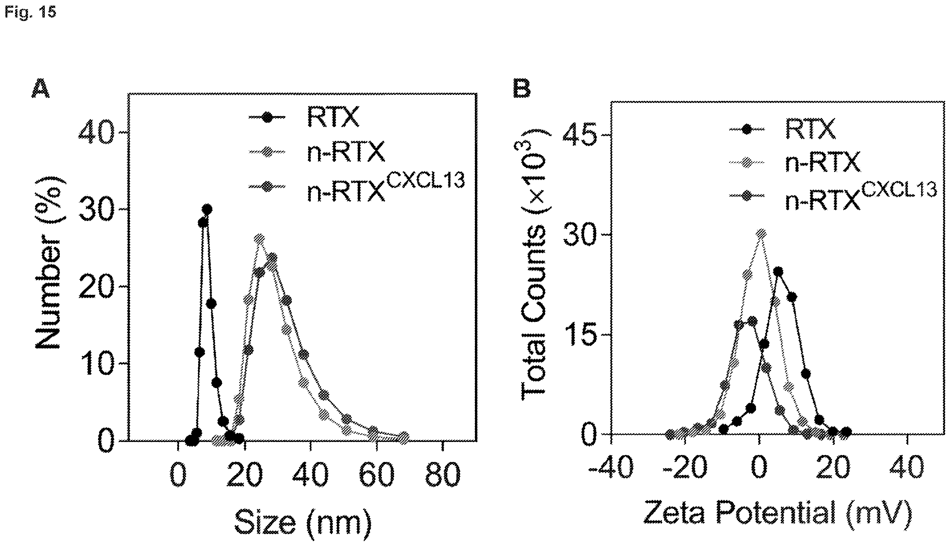

**Figures 15A-15B provide data showing that CXCL13 conjugation does not affect both size and surface charge of n-RTX.** The distribution of size and zeta potential of RTX, n-RTX, and n-RTXCXCL13 monitored by DLS. Figure 15(A): The size distribution of native RTX, n-RTX, and n-RTXCXCL13 were 8.7 nm, 24.4 nm, and 28.2 nm, respectively. Figure 15(B): The zeta potential of native RTX, n-RTX, and n-RTXCXCL13 were 5.1 mV, 0.5 mV, and -0.8 mV, respectively.

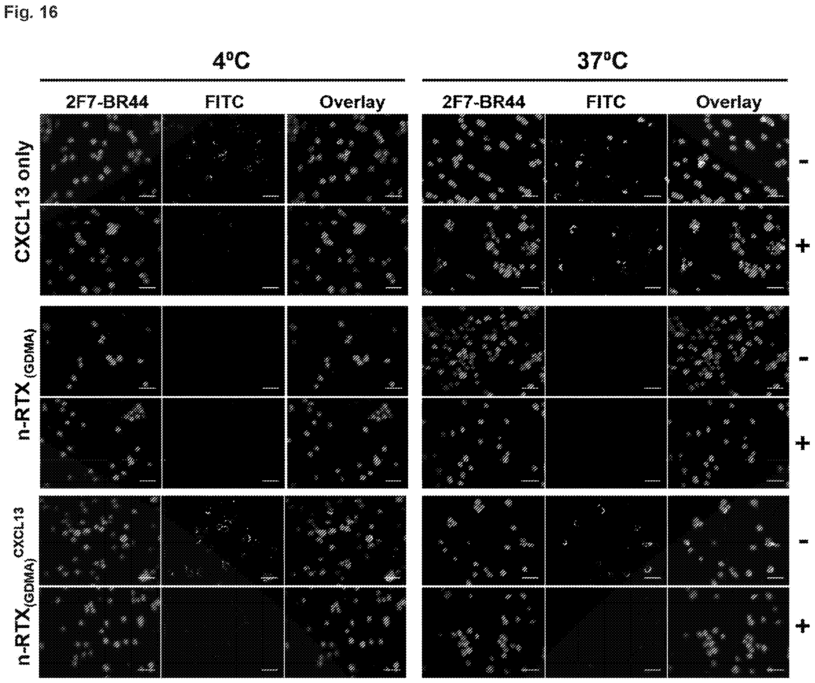

**Figure 16 provides data showing that n-RTX bind to 2F7-BR44 cells in a CXCL13-dependent manner, but do not internalize into the cells.** FITC-labelled CXCL13, n-RTX(GDMA) and n-RTX(GDMA)CXCL13 (0.1 mg/mL) were incubated with 2F7-BR44 cells in 96-well plates (105 cells) for 4 hours at 4 °C and 37 °C. Fluorescence images were taken with Leica DMi8 inverted microscope after fixation with 2% formaldehyde in PBS solution. The fluorescence of FITC was quenched by trypan blue (0.1 %) before fixation. Bar=30 $\mu$m. -: no trypan blue, + quenched by trypan blue.

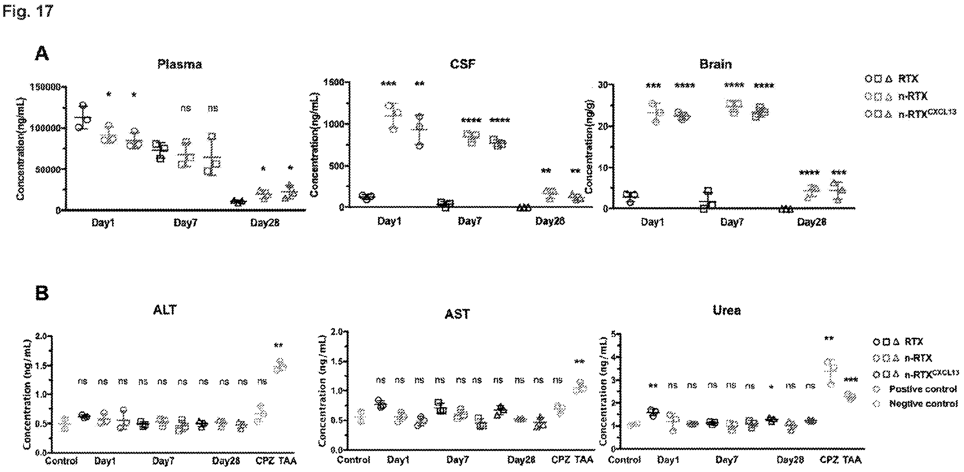

**Figures 17A-17B provide data showing that CXCL13 conjugation does not affect pharmacokinetics of n-RTX nor mediate acute toxicity in mice.** Pharmacokinetics in Figure 17(A) and acute toxicity in Figure 17(B) of RTX nanocapsules with or without CXCL13 conjugation were assessed in a murine model. B6 mice were randomly separated into three groups (n=3) and administered with 20mg/kg of native RTX, n-RTX, or n-RTXCXCL13 via retro-orbital vein. Plasma, CSF, and perfused brain homogenates were collected on days 1, 7, and 28. Figure 17(A): Concentrations of free RTX were measured by ELISA. Figure 17(B): Acute toxicity of RTX nanocapsule with or without CXCL13 conjugation was evaluated by measuring levels of alanine transaminase (ALT), aspartate aminotransferase (AST), and urea nitrogen (Urea) in mouse plasma. Chlorpromazine Hydrochloride (CPZ, 100mg/kg) or Thioacetamide (TAA, 100mg/kg) were used as inducers for kidney and liver toxicities. Two mice without any injections were used as a negative control. Data are shown means $\pm$ SDs of triplicate measurements. The statistical significance was calculated to native RTX group (A) and control group (B) using a p-value, *: P value< 0.0332; **: P values <0.0021; ***: P value<0.0002, ****: P value<0.0001; ns: not significant.

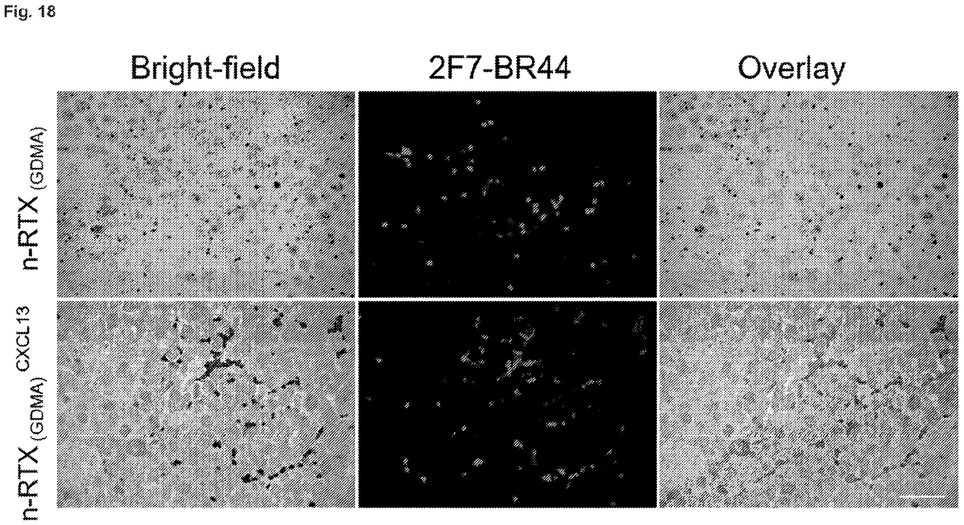

**Figure 18 provides data showing that CXCL13-conjugated n-RTX colocalize with 2F7-BR44 cells in the kidney.** 2F7-BR44 cells were injected via tail vein (2x106/animal). Gold-nanoparticle-labelled n-RTX(GDMA) or n-RTX(GDMA)CXCL13 were administered via retro-orbital vein at week 3 post-2F7-BR44 cell injection. Kidneys were obtained 1 day after administration and processed for IHC analysis. Gold nanoparticles in paraffin sections were analyzed following silver enhancement, which present as black depositions. 2F7-BR44 cells were visualized by mStrawberry fluorescence. Bar=25 $\mu$m.

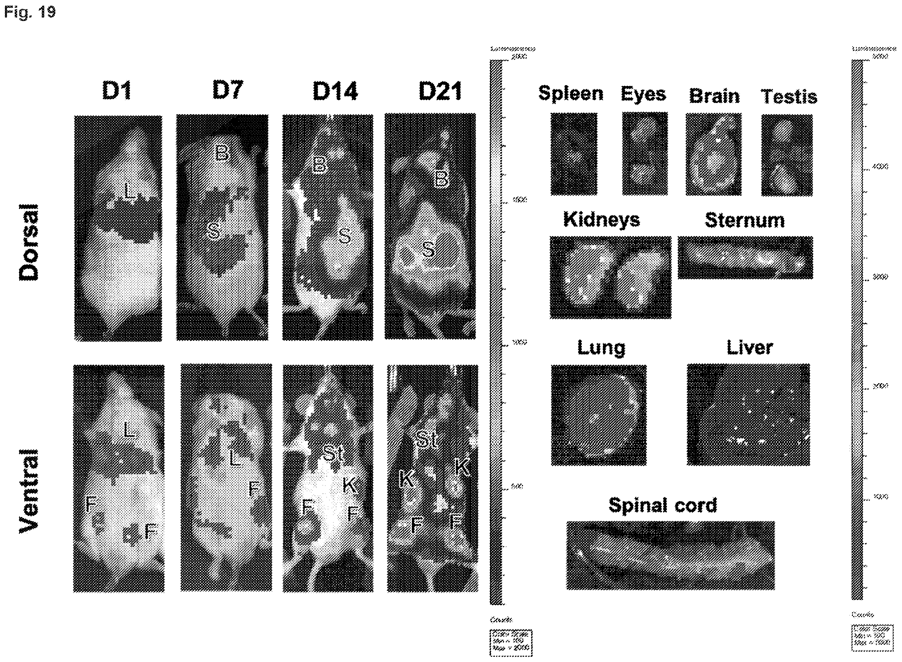

**Figure 19 provides data showing a series of in vivo bioluminescent images of a representative xenograft NSG mouse engrafted with luciferase-labeled 2F7-BR44 cells.** 2F7-BR44 expressing firefly luciferase (2F7-BR44-Luc) cells were transplanted to NSG mice (n=6) via tail vein (2x106/animal). Representative bioluminescent images from the same animal were taken on IVIS Lumina II In vivo imaging system at days 1, 7, 14, and 21 post-transplantation. B: brain, S: spinal cord, St: sternum, K: kidney, F: femur, and L: lung. Organ images were taken at week 3 post-2F7-BR44-Luc cell injection.

## DETAILED DESCRIPTION OF THE INVENTION

Many of the techniques and procedures described or referenced herein are well understood and commonly employed using conventional methodology by those skilled in the art. In the description of the preferred embodiment, reference may be made to the accompanying drawings which form a part hereof, and in which is shown by way of illustration a specific embodiment in which the invention may be practiced. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

CNS metastases are a major cause of cancer deaths with few therapeutic options for treatment. Monoclonal antibody-based therapy is one of the most successful therapeutic strategies for cancer; however, its efficacy is limited against CNS metastases due to insufficient CNS delivery. Here, we show significantly improved antibody delivery to the CNS using novel timed-release nanocapsules that encapsulate individual antibodies within a thin layer of crosslinked phosphorylcholine polymer and gradually release cargo over time through hydrolysable crosslinkers. Surprisingly, a single course of rituximab (RTX) nanocapsule treatment elevates RTX levels in the CNS by nearly 10-fold compared to native RTX. We improved control of CNS metastases in a murine xenograft model of non-Hodgkin lymphoma; moreover, using a xenograft humanized BLT mouse model, lymphomas were eliminated with a single course of RTX nanocapsule treatment. This approach is useful for treatment of cancers with CNS metastases and is generalizable for delivery of any antibody to the CNS.

As discussed in detail below, embodiments of the invention are based upon, for example, the unexpected discovery that certain types of nanocapsules can be used to elevate levels of intravenously administered therapeutic antibody in the CNS by nearly 10-fold by facilitating crossing of the blood brain barrier. Embodiments of the invention include compositions of matter, as well as methods for making and using such compositions. For example, embodiments of the invention include compositions of matter as defined in claim 1 that include a polypeptide cargo and a polymeric network selectively configured to form a polymer shell around the cargo, using, for example, neutral monomers with zwitterionic properties, and 2-methacryloyloxyethyl phosphorylcholine as well as selected hydrolysable crosslinking agents/moieties that are coupled to the polymers that form the nanocapsule that surrounds the polypeptide cargo. In such embodiments of the invention, the nanocapsule is formed in situ on the polypeptide cargo; the polymeric network and the hydrolysable crosslinking moiety and their relative amounts are disposed in a three-dimensional architecture so that: the nanocapsule crosses blood brain barriers to deliver nanocapsules in a bloodstream into a central nervous system; and the hydrolysable crosslinking moiety is cleaved in the central nervous system so as to release the polypeptide cargo from the nanocapsule.

Optionally such compositons of matter include a plurality of different nanocapsules, wherein specific constituents and/or ratios of the nanocapsule components (e.g. degradable crosslinkers and the like) in the plurality of nanocapsules are selected and disposed in a three-dimensional architecture in the nanocapsule so that the plurality of nanocapsules release the polypeptide cargos at different rates of time. In certain embodiments of the invention, the constituents and ratios of the shell components, the polypeptide cargo antibody), the polymeric network and the hydrolysable crosslinking moiety are disposed in a three-dimensional architecture so that at least 2, 5 or 9-fold more protein cargo crosses blood brain barriers as compared with a control protein cargo in the absence of the nanocapsule.

A specific embodiment of the invention as described in claim 1 is a composition of matter comprising an antibody; a polymeric network formed from 2-methacryloyloxyethyl phosphorylcholine and configured to form a nanocapsule that encapsulates the antibody; a hydrolysable crosslinking moiety comprising a glycerol dimethacrylate and/or a Poly(DL-lactide)-b-Poly(ethylene glycol)-b-Poly(DL-lactide)-diacrylate triblock copolymer coupled to polymers forming the polymeric network; and a targeting agent comprising CXCL13 coupled to the polymeric network. In this embodiment of the invention, the nanocapsule is formed in situ on the polypeptide cargo; and the nanocapsule constituents and ratios of the polypeptide cargo, the polymeric network, the hydrolysable crosslinking moiety and the targeting agent are disposed in a three-dimensional architecture so that the nanocapsule crosses blood brain barriers to deliver nanocapsules in a bloodstream into a central nervous system such that at least about 2, 5 or 10 fold more antibody that is disposed in the nanocapsule crosses blood brain barriers as compared with the antibody in the absence of the nanocapsule; and the hydrolysable crosslinking moiety is cleaved in the central nervous system so as to release the antibody from the nanocapsule.

Another embodiment of the invention is a method of making a polypeptide cargo antibody (e.g. an antibody selected to target cancerous cells) encapsulated by a polymeric network as defined in claim 2. Typically, the method comprises performing an in situ polymerization process on monomers comprising a 2-methacryloyloxyethyl phosphorylcholine moiety) that form electrostatic interactions with the polypeptide cargo so as to form a nanocapsule that encapsulates the polypeptide cargo; coupling polymers in the polymeric network with one or more hydrolysable crosslinking moieties, wherein the hydrolysable crosslinking moieties are selected to cleaved in the central nervous system so as to release the polypeptide cargo from the nanocapsule; wherein the polymeric network is coupled to one or more targeting agents, wherein the targeting agents CXCL13 are selected to bind to targets present in tissues of the central nervous system (e.g. a polypeptide comprising a CXCL13 motif). In such embodiments, the nanocapsule constituents and ratios of the polypeptide cargo, the polymeric network, the hydrolysable crosslinking moiety and the targeting agent are selected to allow the nanocapsule to cross blood brain barriers to deliver protein cargos into a central nervous system; and also the hydrolysable crosslinking moiety to be cleaved in the central nervous system so as to release the polypeptide cargo from the nanocapsule.

Yet another embodiment of the invention is a method (not claimed) of selectively delivering a therapeutic agent to the central nervous system of an individual or use in delivering a therapeutic agent, the method or use comprising intravenously administering a nanocapsule composition disclosed herein to the individual; and then allowing the composition to cross the blood brain barrier of the individual into the central nervous system. The composition comprises an antibody, a polymeric network formed from 2-methacryloyloxyethyl phosphorylcholine and configured to form a nanocapsule that encapsulates the antibody, a hydrolysable crosslinking moiety comprising a glycerol dimethacrylate and/or a Poly(DL-lactide)-b-Poly(ethylene glycol)-b-Poly(DL-lactide)-diacrylate triblock copolymer coupled to polymers forming the polymeric network, and a targeting agent CXCL13(e.g. one that targets diseased tissue within the central nervous system) coupled to the polymeric network. In such embodiments of the invention, the nanocapsule is formed *in situ* on the polypeptide cargo antibody, the nanocapsule constituents and ratios of the antibody, the polymeric network, the hydrolysable crosslinking moiety and the targeting agent are disposed in a three-dimensional architecture so that the nanocapsule crosses blood brain barriers to deliver nanocapsules present in a bloodstream into a central nervous system such that at least 2, 3, 4 or 5 or fold more antibody disposed in nanocapsules crosses blood brain barriers as compared with the antibody not encapsulated by nanocapsules as is surprisingly observed in working embodiments of the invention (see, e.g. the data shown in Figure 1). Typically in these embodiments, the hydrolysable crosslinking moiety is cleaved in the central nervous system so as to release the antibody from the nanocapsule.

As disclosed herein, embodiments of the invention provide for the effective delivery of mAb to the CNS by encapsulating the anti-CD20 mAb rituximab (RTX) within a thin shell of polymer that can comprise, for example, analogs of choline and acetylcholine receptors (e.g. as targeting agents). This encapsulated RTX, denoted as n-RTX, eliminated lymphoma cells systemically in a xenografted humanized mouse model using an immunodeficient mouse as a recipient of human hematopoietic stem/progenitor cells and fetal thymus more effectively than native RTX; importantly, n-RTX showed notable anti-tumor effect on CNS metastases which is unable to be shown by native RTX.

We further illustrated the properties of n-RTX in immunocompetent animals, rats, and non-human primates (NHPs). Results from these studies show that a single intravenous injection of n-RTX resulted in 10-fold greater levels in the CNS and 2-3-fold greater levels in the lymph nodes of RTX, respectively, than the injection of native RTX in both rats and NHPs. In addition, we demonstrate the enhanced delivery and efficient B-cell depletion in lymphoid organs of NHPs with n-RTX. Moreover, detailed hematological analysis and liver enzyme activity tests indicate n-RTX treatment is safe in NHPs. As this nanocapsule platform can be universally applied to other therapeutic mAbs, it holds great promise for extending mAb therapy to poorly accessible body compartments. See Qin et al., "Enhanced Delivery of Rituximab Into Brain and Lymph Nodes Using Timed-Release Nanocapsules in Non-Human Primates" Nature Biomedical Engineering, page 706-716, 2019, PMID: 31384008.

Further illustrative aspects and embodiments of the invention are described in the following sections.

### Nanocapsules facilitate CNS penetration.

We have developed a nanotechnology platform whereby individual macromolecules are encapsulated within a thin polymer shell formed by *in situ* polymerization of monomers and stabilized by environmentally-responsive crosslinkers (28,29). Like a virion capsid, the polymer shell shields cargo from the environment and confers high resistance to denaturation, proteases, and nucleases, and determines the distribution of nanocapsules. Nanocapsules with polymer shells formed by neutral monomers with zwitterionic properties, 2-methacryloyloxyethyl phosphorylcholine (MPC), exhibited broad biodistribution (30) and a long half-life (31). MPC is used in contact lenses and tested for use in coronary stents and other medical devices (32,33) and is inert, highly stable, resistant to protein adsorption, and lacks immunogenicity. We synthesized RTX nanocapsules with MPC monomers and glycerol dimethacrylate (GDMA) crosslinkers, which are efficiently degraded under acidic conditions but very slowly under physiological conditions (termed n-RTX$_{(GDMA)}$).

We first evaluated the biodistribution and brain delivery efficiency of our nanoparticles without release of the RTX cargo under physiologic conditions using n-RTX$_{(GDMA)}$ (Fig.7). Biodistribution of the nanocapsules was assayed by enzyme-linked immunosorbent assay (ELISA) after releasing RTX by acid treatment (pH5.4) at 4 $^0$C for overnight *ex vivo*. Higher levels of n-RTX$_{(GDMA)}$ were present in plasma by day 7. Importantly, more RTX released from n-RTX$_{(GDMA)}$ was observed in the brain and cerebrospinal fluid (CSF). Levels of released RTX in CSF were 1.7-5.5% the levels in plasma (5.5% on day 1, 1.7 % on day 7, and 3% on day 21); native RTX was undetectable in CSF at all three points. This is consistent with the relative inability of antibodies to cross the BBB (4).

To achieve proper release of mAb for therapeutic treatment, nanocapsules were designed to release cargo over time. This is accomplished by securing the polymer shell with mixed crosslinkers that are hydrolyzed gradually under physiological conditions (Fig.1A). MPC monomers are enriched around the surface of individual RTX molecules through electrostatic interactions, then hydrolysable crosslinker (Poly(DL-lactide)-b-Poly(ethylene glycol)-b-Poly(DL-lactide)-diacrylate triblock copolymers (PLA-PEG-PLA) and slowly-hydrolysable crosslinker GDMA under physiological conditions are associated by hydrogen bonding. Subsequent polymerization in an aqueous solution wraps each molecule with a thin shell of polymer through *in-situ* free-radical polymerization. When PLA-PEG-PLA is hydrolyzed by body fluids, the nanocapsules dissociate and release encapsulated RTX. These nanocapsules have a relatively uniform, small diameter of 20-30 nm as measured by transmission electron microscope (TEM) (Fig.8) and consist of a single-RTX molecule core-shell structure (Fig.9). To sustain timed release in plasma at neutral pH, we mixed GDMA with PLA-PEG-PLA to achieve different release rates. Depending on the relative ratios of PLA-PEG-PLA (100%, 50%, 30%, and 10%), these nanocapsules released RTX at different rates under physiological conditions (Fig.1B): RTX nanocapsules with 50% PLA-PEG-PLA crosslinker (termed n-RTX) enabled release to occur gradually over 7 days. Importantly, lower pH (pH 5.5-6.0) allowed accelerated RTX release (Fig. 1C), which would facilitate quicker release of RTX in the lower pH tumor microenvironment (34,35) than in the bloodstream or healthy tissues.

We next examined the kinetics of RTX released from nanocapsules with 50% PLA-PEG-PLA crosslinker in B6 mice (Fig.1D,E). Mice were injected with a single intravenous dose of either n-RTX or native RTX. Except for Day 1, where RTX was slightly lower in the n-RTX group, there were comparable levels in plasma through Day 28. In striking contrast, 8 to 10-fold enhancements of free RTX levels were surprisingly observed in the CNS and brain tissue when delivered by n-RTX relative to native RTX. Mice treated with n-RTX had detectable levels of RTX in the CNS and brain for up to 4 weeks. Similar results were also confirmed in rats. Immunohistochemical staining of rat brains treated with n-RTX showed normal microglia and astrocyte morphology (Fig. 10) (36,37). No significant elevation of ionized calcium-binding adapter molecule 1 (Iba1) in microglial cells nor glial fibrillary acidic proteins (GFAP) in astrocytes of rat brains treated with nanocapsules were observed compared to untreated control animals (Fig.10B,C). These results provide proof of concept for penetration, release, maintenance, and safety of mAb nanocapsules in both plasma and CNS.

### Encapsulated RTX effectively controls CNS lymphoma formation.

Next, we compared anti-lymphoma efficacy of native RTX and n-RTX in a xenograft murine model. We first established a human NHL xenograft murine model that can consistently form CNS lymphomas with 2F7 cells, a cell line derived from AIDS-associated B-cell NHL (38). These 2F7 cells were marked with an mStrawberry reporter gene by a lentiviral vector. Individual sub-clones were tested for their ability to metastasize into the CNS. Two out of ten sub-lines formed CNS lymphomas within 7 weeks after intraperitoneal injection. Lymphoma cells were isolated from the brain of one of these sub-lines and re-adapted to cell culture. The resulting cell line, termed "2F7-BR44," forms lymphomas in CNS in 100% of mice within 7 weeks following intraperitoneal injection or 1-2 weeks following tail vein injection of $2 \times 10^6$ cells (Fig.11). 2F7-BR44 cells maintain expression of four pan B cell markers (CD19, CD20, CD22, and CXCR5) at levels comparable to the parental 2F7 cells (Fig. 12A). The cells are sensitive to apoptosis mediated by RTX, comparable to the parental cell line, and also sensitive to complement *in vitro* (Fig. 12B,C).

Lymphoma burden and therapeutic efficacy were quantified by evaluating the percentage of mStrawberry+ 2F7-BR44 cells in different tissues. CNS metastases generally arise later than those in systemic organs; without treatment, animals usually die from systemic complications. Mice with lymphoma show apparent signs of lymphoma burden such as weight loss, pale skin, anemia, and decreased activity. Once the lymphoma burden in CNS increases up to ~15%, as quantified by the percentage of mStrawberry+ in single cell fraction obtained from whole brain tissues following perfusion, mice show hind leg paralysis. Native RTX treatment significantly extended survival of mice engrafted with 2F7-BR44 cells (Fig. 13B) and efficiently reduced lymphoma burden in bone marrow as well as to a lesser extent in spleen, but was ineffective in brain as well as lymph nodes (Fig. 13C).

We next compared the efficacy of native RTX versus n-RTX in controlling lymphoma formation in the brain. n-RTX improved the therapeutic efficacy of RTX against CNS metastasis in the NHL murine model; in contrast, untreated animals required euthanization at week 3.5-4 due to severe anemia (Fig.2A). Treatment significantly improved mouse life span: native RTX treated mice survived up to week 5, whereas half of the n-RTX treated mice showed no symptoms of lymphoma formation in brain until the end time points (week 5.5). Both native RTX and n-RTX capably suppressed lymphoma formation in bone marrow. In contrast, n-RTX significantly reduced lymphoma burden in the brain, whereas native RTX had no effect despite effective systemic control (Fig.2B). The correlation between controlled lymphoma growth and heightened RTX concentration illustrated by these results further demonstrates that RTX delivered via nanocapsules exerts control over CNS metastases.

### Nanocapsules conjugated with CXCL13 can target lymphoma cells by binding to CXCR5.

Ligands can be readily conjugated to the surface of nanocapsules to redirect them to specific targets; thus, targeting nanocapsules to lymphoma cells is predicted to increase RTX concentrations at the site of the lymphoma, enhancing potency and specificity of activity. To demonstrate the potential of ligand-mediated lymphoma targeting, we selected CXCL13: a chemokine belonging to the CXC family that interacts with CXCR5, a receptor expressed on mature B cells (39,40), and is associated with NHLs of B cell origin (41-43). In malignancies, CXCL13 has suspected involvement in metastasis of lymphoma cells (44,45); therefore, to assure accurate modelling of the metastatic environment, we evaluated CXCR5 expression in 2F7-BR44 cells and confirmed that they showed a similar level of CXCR5 expression to that of parental 2F7 cells (Fig.12A). We then conjugated CXCL13 to the surface of nanocapsules at a molar ratio of approximately 1:1 (Fig. 14, n-RTX$^{CXCL13}$). This conjugation did not induce significant change on either particle size or surface charge of n-RTX (Fig.1 5A,B).

To evaluate the binding of CXCL13-conjugated nanocapsules, EGFP was used as a model protein for nanocapsule synthesis with slowly hydrolysable GDMA crosslinkers under physiological conditions (n-EGFP$_{(GDMA)}$ and n-EGFP$_{(GDMA)}$$^{CXCL13}$). We found that CXCL13 conjugation improved the specific binding of nanocapsules on 2F7-BR44 cells (Fig.3B, 2F7) while minimizing non-specific binding on non-targeted cells (Fig.3B, Jurkat); this specific binding of n-EGFP$_{(GDMA)}$$^{CXCL13}$ on 2F7-BR44 cells was further confirmed by flow cytometry (Fig.3C). Importantly, CXCL13 conjugation did not change the surface properties of MPC nanocapsules, maintaining resistance to cellular uptake. Similarly to n-RTX$_{(GDMA)}$, n-RTX$_{(GDMA)}$$^{CXCL13}$ bound to 2F7-BR44 cells but was not internalized (Fig.16). Biodistribution data showed that both n-RTX$_{(GDMA)}$ and n-RTX$_{(GDMA)}$$^{CXCL13}$ systemically distributed in mice (Fig.3D), were delivered with uniform efficiency within the brain, and exhibited decreased accumulation in the liver, kidney, and lung-tissues known for showing non-specific accumulation of antibody (Fig.3E,F).

We next demonstrated the enhanced effectiveness of n-RTX conjugated with CXCL13 (n-RTX$^{CXCL13}$) in controlling lymphoma growth in a xenograft murine model. Compared to native RTX, there was significant improvement in the kinetics of RTX delivery released from both n-RTX and n-RTX$^{CXCL13}$ into CSF and brain; minimal differences were noted between the two kinds of nanocapsules (Fig.17A). Moreover, there were no obvious liver or kidney toxicities over 4 weeks of nanocapsule treatment (Fig.17B). Importantly, compared to native RTX and n-RTX-treated animals, mice treated with n-RTX$^{CXCL13}$ showed improved survival rate (Fig.4A) and minimal CNS lymphoma formation until the end time points (Fig.4B). Greater numbers of n-RTX$^{CXCL13}$ were observed in areas of lymphoma growth (Fig.4C,D), indicating that n-RTX$^{CXCL13}$ preferentially locates with 2F7-BR44 lymphoma in the brain. Besides lymphomas in brain, renal lymphoma and intraocular lymphoma were also observed in this NHL murine model. Unlike native RTX treatment, intraocular lymphoma was prevented by treatment with n-RTX; however, nodules still formed on kidneys (Fig.4E). n-RTX$^{CXCL13}$ showed clear colocalization with 2F7-BR44 cells in the kidney (Fig.18) and exerted superior anti-lymphoma activity in both locations compared to native RTX or n-RTX (Fig.4E).

***Nanocapsules conjugated with CXCL13 improved therapeutic efficacy at different stages of lymphoma progression.***

To facilitate longitudinal analysis of lymphoma location and progression, the NHL mouse model was further adapted for optical imaging by marking 2F7-BR44 cells with a lentiviral vector expressing firefly luciferase (2F7-BR44-Luc). Following injection of D-luciferin, the substrate of luciferase, *in vivo* distributions and growth of 2F7-BR44-Luc cells were monitored by *in vivo* bioluminescence imaging over time (Fig. 19). Bioluminescence imaging in the NHL xenograft murine model indicated that 2F7-BR44 cells initially migrated into the lungs after tail vein injection, followed by redistribution to CNS (brain and spinal cord) and bone marrow (BM) from sternum and femur within the first week. At week 2, signal intensity from lymphomas in the brain, spinal cord, sternum, femur, and kidneys increased.

Based upon the kinetics of lymphoma formation, we conducted studies in which treatment was initiated after obvious lymphoma formation. Mice were treated with native RTX or n-RTX$^{CXCL13}$ at different times: Week 1 (Group I) and Week 2 (Group II) post-injection of 2F7-BR44-Luc cells. Treatment by n-RTX$^{CXCL13}$ in both groups resulted in significant control of lymphoma burden relative to native RTX treatment, measured by bioluminescence per a defined area (Fig.5A,B). Survival of lymphoma-bearing mice was significantly extended for 1-2 weeks by n-RTX$^{CXCL13}$ compared to native RTX in both Group I and II (Fig.5C,D). To quantify the difference in therapeutic efficacy between native RTX and n-RTX$^{CXCL13}$, bioluminescence intensity (BLI) from 2F7-BR44-Luc cells in the whole body was compared every week after treatment (Fig.5E,F). In Group I, whole body therapeutic efficacy was similar in the first week post-treatment across both treatment types but was significantly improved by n-RTX$^{CXCL13}$ later, suggesting more effective control of lymphoma burden by n-RTX$^{CXCL13}$. In Group II, there was a significantly greater effect of n-RTX$^{CXCL13}$ treatment, but less than in mice treated in Group I. To assess the impact upon CNS lymphomas, BLIs from the head area were quantified (Fig.5G,H). Treatment with n-RTX$^{CXCL13}$ notably slowed and controlled CNS lymphoma burden in both groups. In Group II, where lymphomas had already formed in CNS before treatment, all mice with n-RTX$^{CXCL13}$ showed a decrease in lymphoma burden at 1 week following treatment.

***Nanocapsules conjugated with CXCL13 eliminated lymphoma in xenograft humanized BLT mice.***

The above NHL xenograft murine model uses immunodeficient mice in which the killing of 2F7-BR44-Luc cells is likely to be primarily, if not exclusively, by induction of apoptosis. RTX is known to induce anti-lymphoma killing through multiple mechanisms in addition to apoptosis, including ADCC and CDC (26). Thus, the efficacy of RTX against CNS lymphomas in the NHL xenograft model is highly limited (46). We further evaluated n-RTX[CXCL13] in a humanized BLT (bone marrow/liver/thymus) murine model wherein human T cells, B cells, natural killer (NK) cells, and macrophages reconstitute; of these populations, both NK cells and macrophages can induce ADCC. Humanized BLT mice develop notably human NK and macrophage populations in tissues, including the brain (47-50). Consistent with a published study (51), we found substantial macrophage repopulation in the brains of these mice. Humanized BLT mice were treated with native RTX or n-RTX[CXCL13] at week 1 post-injection of 2F7-BR44-Luc cells and monitored for lymphoma growth for 12 weeks while unrelated antibody Herceptin (anti-human epidermal growth factor receptor (HER) 2) and n-HER[CXCL13] were included as negative controls. Native RTX treatment showed effective therapeutic efficacy throughout the body except in brain, which resulted in paralysis on weeks 4-8; additionally, neither native HER nor n-HER[CXCL13] treatment showed any therapeutic effect (Fig.6A). In contrast, n-RTX[CXCL13] completely eliminated lymphomas in mice and initiated regression of CNS lymphomas, with no relapse observed even until the endpoint at week 13. Survival of lymphoma-bearing mice was significantly extended by n-RTX[CXCL13] treatment (Fig.6B). The clearance of lymphomas was confirmed by flow cytometry of tissues (Fig.6C).

## Discussion

We demonstrate that a single-course treatment of RTX encapsulated within an MPC-based nanocapsule designed to administer mAb by timed release results in CNS RTX concentrations up to 10-fold higher than native antibody and yields detectable levels for at least 4 weeks in a murine model. Using an NHL xenograft murine model, better penetration into the CNS allows control of CNS lymphoma formation. Three basic components were tuned to increase anti-lymphoma effects of RTX: 1) a polymer shell which allows longer systemic circulation and CNS penetration, 2) crosslinkers which stabilize the polymer shell and release mAb through timed hydrolysis, and 3) CXCL13 conjugated to the surface enabling targeting to CXCR5-expressing lymphoma cells. CNS penetration of the nanocapsules appears to be mediated by binding of choline and acetylcholine analogues of the nanocapsule polymer shell to choline transporters and acetylcholine receptors.

Enhanced RTX levels in the CNS could act to control local lymphoma growth through various effector mechanisms. Both CDC and ADCC function in the CNS (52-55), but are likely limited due to low antibody levels. In the NSG xenograft murine model, induction of apoptosis is likely to be the major mechanism for lymphoma cell elimination since complement components are absent, and it is unclear whether murine microglia may contribute to ADCC activity (53). With limited effector functions, only partial control was achieved in xenografted NSG mice. By repeating studies in humanized BLT mice wherein human macrophages and NK cells differentiate, we demonstrated complete elimination of lymphomas both systemically and in the CNS. We suspect that the differences between these two murine models exist due to the presence of ADCC in the humanized BLT mice, which is absent in the standard xenograft model using NSG mice. Future studies will better elucidate the mechanisms responsible for lymphoma control and clearance.

The results of these studies are applicable to other therapeutic mAbs wherein CNS penetration is limited. For example, breast cancer patients controlled systemically by Herceptin often relapse with CNS metastases that are resistant to mAb therapy (7,56). By bypassing the BBB through intrathecal or intraventricular administration, mAb therapy for lymphoma and breast cancer has shown some effectiveness against CNS metastases (7,57). Our results provide a potential non-invasive alternative treatment of CNS lymphomas as well as prophylactic use of the nanocapsule against CNS metastases. Since the biodistribution properties of the mAb are conferred by the nanocapsule, not cargo, any mAb (or protein) can be readily substituted. Anti-phosphorylcholine autoantibodies have been reported in mice (58) and humans (59), and could potentially affect *in vivo* dynamics of nanocapsules generated with MPC, though we do not observe more rapid clearance relative to native mAb. We anticipate that further studies on biodistribution and optimization of formulations through engineering design of polymers, crosslinkers, and targeting ligands will further improve CNS delivery and therapeutic efficacy for CNS diseases.

## METHODS

**Synthesis of nanocapsules.** Nanocapsules were synthesized with optimized modication for antibodies based on our previous reports (31). Proteins (RTX or EGFP) were encapsulated via *in situ* polymerization at room temperature using MPC as the monomer, PLA-PEG-PLA and/or GDMA as the crosslinker, and ammonium persulfate and tetramethylethylenediamine as the initiator. Synthesized nanocapsules were dialyzed against PBS, and purified by passing through a hydrophobic interaction column (Phenyl-Sepharose 4BCL).

**Biodistribution studies.** Biodistribution of native RTX and n-RTX was determined by monitoring the free RTX concentration in animal body fluids and perfused tissue homogenates. Briefly, C57BL/6 mice were randomly divided into two groups and retro-orbitally injected at a doseage of 20 mg/kg/mouse, which dose has been reported to be effective in an NHL xenograft murine model (60). CSF was collected from a mouse under anesthesia by Ketamine and Xylazine (100 mg/kg each). After CSF collection, this mouse was perfused with cold phosphate-buffered saline (PBS), euthanized, and organs were harvested. All perfused tissues were homogenized by vortexing with ceramic beads in PBS containing protease inhibitor cocktail.

**Non-Hodgkin Lymphoma (NHL) mouse model with CNS metastases.** Animal research described in the study was conducted under UCLA's Chancellor's Animal Research Committee (Institutional Animal Care and Use Committee [IACUC]) in accordance with guidelines for housing and care of laboratory animals of the National Institutes of Health (NIH) and the Association for the Assessment and Accreditation of Laboratory Animal Care (A ALAC) International 2F7 cells were first marked with a fluorescent reporter gene by a lentiviral vector expressing mStrawberry. The 2F7 cells were then sub-cloned and tested individually for ability to metastasize into CNS. We selected clone 44 out of 10 clones, which showed a stable brain metastatic ability. An NSG mouse received $2\times10^6$ mStrawberry+ 2F7 clone 44 cells via intraperitoneal injection and showed brain metastasis 7 weeks post-injection (see Fig.11A). Lymphoma cells were isolated from the brain metastatic site and adapted to cell culture to establish the 2F7-BR44 cell line. $2\times10^6$ of 2F7-BR44 cells were injected into NSG mice via lateral tail vein to establish an NHL xenograft murine model with CNS metastases.

**MAb detection by ELISA.** The concentration of RTX in animal body fluids (CSF and plasma) and tissue homogenates was measured by ELISA against RTX. 96-well plates were coated with 1 μg/mL of anti-RTX antibody (diluted in sodium carbonate-bicarbonate buffer), followed by blocking with 1% BSA/PBS for 2 hours at room temperature. Diluted RTX in PBST (0.1% Tween/PBS) from 0 to 500 ng/mL were then added and incubated for an hour at room temperature to obtain calibration curves. Animal body fluids and tissue homogenates containing encapsulated RTX in non-degradable nanocapsules were treated with 100 mM sodium acetate buffer (pH 5.4) at 4°C overnight, then used for ELISA measurement. Free RTX released from n-RTX was directly measured with animal body fluids and tissue homogenates. All animal samples were added to the well and incubated for an hour at room temperature. After five-times wash with PBST, peroxidase-conjugated anti-human Fc antibody was added and incubated for an additional hour at room temperature. The substrate 3,3',5,5'-Tetramethylbenzidine (TMB) solution was added and incubated until the appropriate color had developed. The reaction was stopped and absorbance at 450 nm was measured with a microplate reader.

**Anti-lymphoma efficacy of RTX in the NHL xenograft murine model with CNS metastases.** 2F7-BR44 cells ($2\times10^6$/animal) were injected into NSG mice via tail vein. Five days after 2F7-BR44 cell injection unless otherwise stated, mice were treated with a single course of native RTX, n-RTX or n-RTX conjugated with CXCL13 (n-RTX$^{CXCL13}$) via retro-orbital vein injection (4mg/kg/day for 5 days). Mice were sacrificed when in critical condition due to lymphoma burden or at the end time points decided in experiment design. The mice were perfused with cold PBS, euthanized, and organs were harvested for single-cell isolation from tissues. Cells from target tissues were stained with anti-human CD45 and CD19, then analyzed by flow cytometry.

*In vivo* **imaging to monitor lymphoma progression.** 2F7-BR44 cells were gene marked with a lentiviral vector expressing firefly luciferase, then luciferase-expressing cells were selected by a week of Zeocin treatment (200 μg/ml) (2F7-BR44-Luc). 2F7-BR44-Luc cells ($2\times10^6$/animal) were injected into NSG mice or BLT humanized mice via tail vein. Humanized mice were prepared as previously described with modifications (61-63). Six-week-old NSG mice were administered Busulfan (35 mg/kg) intraperitoneally. Twenty-four hours later, the mice were implanted with a portion of human fetal thymus combined with fetal liver derived CD34+ cells solidified in Matrigel under the kidney capsule and also via retro-orbital vein injection. After the human blood cell reconstitution in peripheral blood, 2F7-BR44 cells ($2\times10^6$/animal) were injected into humanized BLT mice via tail vein to establish xenograft humanized BLT mice. Lymphoma formation was monitored by *in vivo* bioimaging using the IVIS Lumina II in vivo imaging system (PerkinElmer, Waltham, MA). *In vivo* bioluminescence imaging was performed following subcutaneous injection of 4.5 mg D-luciferin (Pierce, Woodland Hills, CA). Mice were imaged at the signal plateau (10 minutes post-D-luciferin injection) under isoflurane anesthesia. Lymphoma burden was quantified as the total photon flux per second within a region of interest (ROI) (whole body or head area) of the mouse; the ROIs were identically sized for all measurements. Sensitivity settings were adjusted at each time point to maintain 250-5000 counts per pixel for humanized mice and 500-5000 counts per pixel for NSG mice.

**Statistical analyses.** Results are expressed as mean ± standard deviation (SD). Errors depict SD. Data were analysed by Student's t-test and one way analysis of variance (ANOVA) using the GraphPad Prism (La Jolla, CA). The significance of survival curve was compared with a log-rank (Mantel-Cox) test. P-value was shown in GP style. *: P values <0.0332; **: P values <0.0021;***: P values <0.0002;****: P values <0.0001.

**Materials.** All chemicals were purchased from Sigma-Aldrich (St. Louis, MO) unless otherwise noted. All cell culture reagents were purchased from ThermoFisher Scientific (Waltham, MA) unless otherwise noted. Hydrolysable crosslinker Poly(DL-lactide)-b-Poly(ethylene glycol)-b-Poly(DL-lactide)-diacrylate triblock (PLA-PEG-PLA) was purchased from PolySciTech Akina, Inc (West Lafayette, IN). NHS-PEG4-Azide was purchased from ThermoFisher Scientific. Mono-*sulfo*-N-hydroxy-succinimido nanogold labeling reagent (#2025) and the GoldENhance™ EM kit (#2113) were purchased from Nanoprobes (Yaphank, NY). Capture antibody for ELISA against rituximab (RTX) was purchased from Bio-Rad Laboratories (MCA2260, Hercules, CA). HRP-conjugated goat anti-human IgG Fc for ELISA assay was purchased from ThermoFisher Scientific. Anti-human CD19, anti-human CD20, anti-human CD22, anti-human CD185 (CXCR5), and recombinant CXCL13 were purchased from BioLegend (San Diego, CA). RTX (RITUXAN™: Genentech, San Francisco, CA) and HER (Herceptin®: Genentech) were obtained at the UCLA hospital pharmacy. Anti-glial fibrillary acidic protein (GFAP) (clone GA-5) and anti-ionized calcium binding adaptor molecule 1 (Iba1) (MABN92) antibodies were obtained from Biocare Medical (Pacheco, CA) and EMD Millipore (Darmstadt, Germany), respectively. Anti-Ku80 antibody (clone C48E7, Cell Signaling Technology, Danvers, MA) and rhodamine red-X anti-rabbit IgG (Jackson ImmunoResearch, West Grove, PA) were used for detection of 2F7-BR44 cells in the brain. 4'.6-diamidino-2-phenylindole (DAPI) was purchased from Invitrogen. Antifade mounting media was obtained from Vector laboratories (Burlingame, CA). Mouse aspartate aminotransferase (AST) ELISA kit and mouse alanine transaminase (ALT) ELISA kit were purchased from G-Biosciences (St. Louis, MO), and urea nitrogen (BUN) colorimetric detection kit was purchased from Arbor Assays (Ann Arbor, MI). Matrigel was purchased from BD Biosciences (San Jose, CA).

**Instruments.** TEM images of nanocapsules were obtained on a Philips EM120 TEM (Amsterdam, Netherlands) at 100000x. Fluorescent images of cells were obtained with a Revolve R4 fluorescent microscope, Discover Echo Inc (San Diego, CA). Fluorescent images of brain tissue and tissue sections were obtained with the Maestro™ In-Vivo fluorescence imaging system, CRi, Inc (Dallas, TX) and Leica DMi8 inverted microscope (Buffalo Grove, IL), respectively. Optical images and bioluminescence were collected on IVIS Lumina II In vivo imaging system (PerkinElmer, Inc, Waltham, MA). Cell numbers were acquired via either manual counting with trypan blue dye exclusion under microscope or with the MACSQuant® Analyzer 10 (Miltenyi Biotech, Bergisch Gladbach, Germany). Expression of mStrawberry and cell surface markers were analyzed by BD LSRFortessa (BD Biosciences). UV/VIS spectra were acquired with a GeneSys 6 spectrometer (ThermoFisher Scientific). UV/VIS absorbance was measured by NanoDrop One spectrophotometer (ThermoFisher Scientific). The detection of ELISA was acquired by use of the VersaMax™ Tunable microplate reader (Molecular Devices, San Jose, CA). Dynamic light scattering (DLS) studies of native proteins and nanocapsules were obtained by Zetasizer Nano instrument (Malvern Instruments Ltd., Kingdom).

**Synthesis of RTX nanocapsules.** RTX nanocapsule with mixed crosslinkers of glycerol dimethacrylate (GDMA) and PLA-PEG-PLA at a 1:1 molar ratio (n-RTX) as well as RTX nanocapsules with GDMA (n-RTX$_{(GDMA)}$) were synthesized with optimized modification for antibodies based on our previous reports (1). The n-RTX were synthesized using a volume of 5 mg RTX at 1.5 mg/mL, a specific amount of 2-methacryloyloxyethyl phosphorylcholine (MPC, 40% *m/v* in PBS), PLA-PEG-PLA (10% *m/v* in PBS) and GDMA (10% *m/v* in DMSO) in a molar ratio of RTX:MPC:PLA-PEG-PLA:GDMA=1:12000:500:500. The n-RTX$_{(GDMA)}$ were synthesized using the same amount of RTX, MPC (40% *m/v* in PBS) and GDMA (10% *m/v* in DMSO) at a molar ratio of RTX:MPC:GDMA=1:12000:1000. Radical polymerization from the surface of the protein was then initiated by adding ammonium persulfate (10% *m/v* in PBS, molar ratio to RTX=2000:1) and N,N,N',N'-tetramethylethylenediamine (TEMED, molar ratio to RTX=4000:1) into the reaction vial and kept in ice bath for 2 hours. Finally, dialysis was used to remove extra free monomers and initiators. The free RTX was removed using hydrophobic interaction chromatography (Phenyl Sepharose CL-4B; Sigma Aldrich) as described previously (2).

**Synthesis of nanogold-labeled n-RTX$_{(GDMA)}$.** Native RTX (0.5 mg/mL in PBS) was reacted with mono-*sulfo*-N-hydroxy-succinimido-nanogold at a 2:1 molar ratio in ice bath for 1 hour as published previously (3). Excess nanogold was removed by size-exclusion spin columns. The concentration of nanogold on each protein was determined by the UV/VIS spectrum at 420 nm (the extinction coefficient $\varepsilon420=155,000$ M$^{-1}$cm$^{-1}$) as 1.05 nanogolds per RTX. The nanogold-labeled RTX was then used for nanocapsule synthesis as described above.

**Preparation of nanocapsules conjugated with CXCL13 ligands.** CXCL13 ligands were conjugated on nanocapsules through copper-free click chemistry [4] Dibenzocyclooctyne-S-S-N-hydroxysuccinimidyl ester (DBCO-SS-NHS) in DMSO (11mg/ml) was added into nanocapsule solution at a 10:1 molar ratio. NHS-PEG4-Azide in DMSO (39mg/ml) was added into CXCL13 solution at a 10:1 molar ratio. Both reactions were processed in an ice bath for 1 hour. Free reagents were removed by size-exclusion spin columns. The conjugation between DBCO-modified nanocapsules and azide-modified CXCL13 was reacted at 4°C for 17 hours at a 1:1 molar ratio.

**Determination of the average number of CXCL13 in n-RTX$^{CXCL13}$**. The average number of CXCL13 on each n-RTX$^{CXCL13}$ was assessed by quantifying the amount of reacted DBCO after CXCL13 conjugation. The numbers of DBCO in DBCO-modified nanocapsules and CXCL13 conjugated nanocapsules were determined by the adsorption at 309 nm based on the Beer-Lambert law as

$$Number\ DBCO\ per\ (n-RTX) = \frac{A309}{\varepsilon309 \times Concentration\ of\ n-RTX}$$

wherein A309 is the absorbance of DBCO-modified nanocapsules at 309 nm; $\varepsilon309$ represents the extinction coefficient of DBCO at 309nm ($\varepsilon309= 12,000$M$^{-1}$cm$^{-1}$). **Protein concentration assay.** Protein content in the form of nanocapsules was determined by bicinchoninic acid (BCA) colorimetric protein assay. Briefly, a tartrate buffer (pH11.25) containing 25 mM BCA, 3.2 mM $CuSO_4$, and protein/nanocapsule samples was incubated at 60°C for 30 minutes. After the reaction was cooled to room temperature, the absorbance reading at 562 nm was determined with a UV/Vis spectrometer. BSA was used as a standard.

**TEM and DLS measurements of the nanocapsules.** For TEM imaging of n-RTX, 5 μL of an n-RTX$_{(GDMA)}$ solution (0.2 mg/mL) was dropped onto a carbon-coated copper grid and removed after a 45 second incubation. The grid was rinsed with water three times and stained with 2% uranyl acetate solution. For TEM imaging of nanogold-labeled n-RTX$_{(GDMA)}$, the signal of nanogolds was enhanced by the GoldENhance™ EM kit. The size and zeta potential of the nanocapsules were measured by DLS under the concentration of 0.5 mg/mL.

**Cell culture.** 2F7 cells were transduced with a lentiviral vector encoding mStrawberry under ubiquitin C promoter. Transduced 2F7 cells were flow sorted for mStrawberry expression and sub-cloned to obtain single cell clones. After *in vivo* selection for brain lymphoma formation, 2F7-BR44 cells were used for all *in vitro* and *in vivo* experiments. Jurkat, a T cell line, was marked with a lentiviral vector encoding blue fluorescent protein (BFP). Parental 2F7, 2F7-BR44, and Jurkat cells were cultured in Iscove's Modified Dulbecco's (IMDM) supplemented with 15% fetal bovine serum, 1% GlutaMax and 1% Antibiotic-Antimycotic. 2F7-BR44-Luc cells were established by transducing a lentiviral vector encoding firefly luciferase together with the bleomycin-resistant gene under elongation factor-1 α promoter and selection in 200 μg/ml of Zeocin.

**Cytotoxicity assay.** Anti-lymphoma activity of RTX on parental 2F7 cells and 2F7-BR44 cells was assessed by absolute cell number counting using MACSQuant® Analyzer 10. For the apoptosis test, parental 2F7 cells and 2F7-BR44 were cultured in 24-well plates ($1\times10^6$/mL) for 24 hours in the presence of RTX (20 μg/mL). Same volumes of cell culture were taken to obtain absolute cell numbers on MACSQuant® Analyzer 10. For complement-dependent cytotoxicity (CDC) testing, parental 2F7 cells and 2F7-BR44 cells were cultured in 24-well plates ($1\times10^6$/mL) for 24 hours in the presence of RTX (20 μg/mL) and 20% human serum with no heat inactivation. Cell death percentages were calculated as cell death (%) = 100 × (1-(cell number after RTX treatment/control without RTX)).

**Cell surface marker staining.** Same cell counts of parental 2F7 cells and 2F7-BR44 cells were taken and washed by 2% fetal bovine serum/PBS. Four antibodies specific for B cell surface markers-CD19, CD20, CD22, and CXCR5-were added and stained at 4°C for 30 minutes. Expression levels were assessed by BD LSRFortessa after fixation with 2% formaldehyde in PBS.

**Immunohistochemical (IHC) analyses.** Mouse brain was embedded in paraffin, sectioned at the coronal plane with 4 μm thickness, and Nissl bodies were stained with Cresyl Violet Nissl stain dye by the UCLA Translational Pathology Core Laboratory (TPCL). Slides were further stained with primary anti-human CD20 antibody, followed by HRP-labeled secondary antibody, then incubated with ABC-peroxidase and diaminobenzidine (DAB). Levels of brain damage were evaluated by elevation of two brain damage markers: GFAP for astrocytes, and Iba1 for microglia and macrophages. Sprague Dawley rats were treated with native RTX or n-RTX (15 mg/kg) and brain tissues were processed for IHC analysis as described above.

The biodistribution of native RTX, n-RTX$_{(GDMA)}$, and n-RTX$_{(GDMA)}$$^{CXCL13}$ in mouse brain and kidney tissues was analyzed by light microscopy. Nanogold-labeled RTX, n-RTX$_{(GDMA)}$, and n-RTX$_{(GDMA)}$$^{CXCL13}$ were synthesized and purified with the protocol described above and administrated to mice (20mg/kg). At day 1 post-administration, mice were perfused with PBS to wash out body fluids and blood cells, followed by perfusion with 4% paraformaldehyde for fixation. Brain and kidney tissues were then harvested and embedded in paraffin. Tissue sections of the coronal plane were prepared and signals of nanogold were enhanced by the GoldENhance™ EM kit. 2F7-BR44 lymphoma cells in the brain were visualized by anti-Ku80 staining. Images were taken by Leica DMi8 inverted microscope. Expression levels of each antigen were quantified from three randomly selected slides of each sample with Software ImageJ.

**Specific binding of CXCL13 conjugated nanocapsules on 2F7-BR44 cells.** 2F7-BR44 cells (mStrawberry+, $5\times10^5$ cells) and Jurkat T cells (BFP+, $5\times10^5$ cells) were mixed and suspended in 0.5 mL Opti-MEM medium with 2% fetal bovine serum. EGFP encapsulated with slow-hydrolysable nanocapsule (n-EGFP$_{(GDMA)}$) or CXCL13 conjugated n-EGFP$_{(GDMA)}$ (n-EGFP$_{(GDMA)}$$^{CXCL13}$) were added to culture at a final concentration of 0.1 mg/mL and mixed thoroughly. After incubation at 37 °C for 1 hour, cells were washed three times with PBS then resuspended in 0.3 mL for flow cytometer analysis. For fluorescent microscopic analysis, cells were transferred to Poly-D-Lysine-coated 8 well chambers. The slide was then mounted by a coverslip with aqueous mounting medium and analyzed using a Leica DMi8 inverted fluorescence microscope. All cells were fixed with 2% formaldehyde in PBS for 20 minutes at room temperature before analysis.

**Fluorescence imaging of nanocapsule internalization.** 2F7-BR44 cells (mStrawberry+, $5\times10^5$ cells) were incubated with FITC-labeled native CXCL13, n-RTX$_{(GDMA)}$, and n-RTX$_{(GDMA)}$$^{CXCL13}$ (0.1 mg/mL) at both 4 °C and 37 °C for 4 hours. Cells were then washed with PBS three times and visualized with a fluorescent microscope. To quench the fluorescent signal of FITC on the cell surface, cells were mixed with 2% trypan blue/PBS with a volume ratio of 1:20 before taking images.

**Biodistribution of the nanocapsules.** The biodistributions of native RTX, n-RTX$_{(GDMA)}$, and n-RTX$_{(GDMA)}$$^{CXCL13}$ were analyzed by optical imaging. Native RTX, n-RTX$_{(GDMA)}$, and n-RTX$_{(GDMA)}$$^{CXCL13}$ were fluorescently labeled with carboxytetramethylrhodamine (TAMRA) and administrated to mice (20mg/kg). *In vivo* fluorescence imaging was performed at 1 day post-administration (Ex.=535 nm, Em.=570 nm) by IVIS Lumina II In vivo imaging system. The main organs, including brain, heart, liver, spleen, kidneys and lung, were harvested following perfusion with PBS for *ex vivo* optical imaging.

**References**

1. Chambers, A.F., Groom, A.C. & MacDonald, I.C. Dissemination and growth of cancer cells in metastatic sites. Nat Rev Cancer 2, 563-572 (2002).
2. Aragon-Ching, J.B. & Zujewski, J.A. CNS metastasis: an old problem in a new guise. Clin Cancer Res 13, 1644-1647 (2007).
3. Tosoni, A., Ermani, M. & Brandes, A.A. The pathogenesis and treatment of brain metastases: a comprehensive review. Crit Rev Oncol Hematol 52, 199-215 (2004).
4. Rubenstein, J.L., et al. Rituximab therapy for CNS lymphomas: targeting the leptomeningeal compartment. Blood 101, 466-468 (2003).
5. Zhang, Y. & Pardridge, W.M. Mediated efflux of IgG molecules from brain to blood across the blood-brain barrier. J Neuroimmunol 114, 168-172 (2001).
6. Czyzewski, K., et al. Intrathecal therapy with rituximab in central nervous system involvement of post-transplant lymphoproliferative disorder. Leuk Lymphoma 54, 503-506 (2013).
7. Lu, N.T., et al. Intrathecal trastuzumab: immunotherapy improves the prognosis of leptomeningeal metastases in HER-2+ breast cancer patient. J Immunother Cancer 3, 41 (2015).
8. Cooper, P.R., et al. Efflux of monoclonal antibodies from rat brain by neonatal Fc receptor, FcRn. Brain Res 1534, 13-21 (2013).
9. Rubenstein, J.L., et al. Multicenter phase 1 trial of intraventricular immunochemotherapy in recurrent CNS lymphoma. Blood 121, 745-751 (2013).
10. Rubenstein, J.L., et al. Phase I study of intraventricular administration of rituximab in patients with recurrent CNS and intraocular lymphoma. J Clin Oncol 25, 1350-1356 (2007).
11. Bousquet, G., et al. Intrathecal Trastuzumab Halts Progression of CNS Metastases in Breast Cancer. J Clin Oncol 34, e151-155 (2016).
12. Chen, Y. & Liu, L. Modern methods for delivery of drugs across the blood-brain barrier. Adv Drug Deliv Rev 64, 640-665 (2012).
13. Schroeder, A., et al. Treating metastatic cancer with nanotechnology. Nat Rev Cancer 12, 39-50 (2011).
14. Lu, C.T., et al. Current approaches to enhance CNS delivery of drugs across the brain barriers. Int J Nanomedicine 9, 2241-2257 (2014).
15. Fu, H. & McCarty, D.M. Crossing the blood-brain-barrier with viral vectors. Curr Opin Virol 21, 87-92 (2016).
16. Spencer, B.J. & Verma, I.M. Targeted delivery of proteins across the blood-brain barrier. Proc Natl Acad Sci USA 104, 7594-7599 (2007).
17. Battaglia, L. & Gallarate, M. Lipid nanoparticles: state of the art, new preparation methods and challenges in drug delivery. Expert Opin Drug Deliv 9, 497-508 (2012).
18. Helm, F. & Fricker, G. Liposomal conjugates for drug delivery to the central nervous system. Pharmaceutics 7, 27-42 (2015).
19. Kreuter, J. Drug delivery to the central nervous system by polymeric nanoparticles: what do we know? Adv Drug Deliv Rev 71, 2-14 (2014).
20. Mishra, B., Patel, B.B. & Tiwari, S. Colloidal nanocarriers: a review on formulation technology, types and applications toward targeted drug delivery. Nanomedicine 6, 9-24 (2010).
21. Papasani, M.R., Wang, G. & Hill, R.A. Gold nanoparticles: the importance of physiological principles to devise strategies for targeted drug delivery. Nanomedicine 8, 804-814 (2012).
22. Clark, A.J. & Davis, M.E. Increased brain uptake of targeted nanoparticles by adding an acid-cleavable linkage between transferrin and the nanoparticle core. Proc Natl Acad Sci U S A 112, 12486-12491 (2015).
23. Bharali, D.J., et al. Organically modified silica nanoparticles: a nonviral vector for in vivo gene delivery and expression in the brain. Proc Natl Acad Sci U S A 102, 11539-11544 (2005).
24. Pardridge, W.M. Drug and gene targeting to the brain with molecular Trojan horses. Nat Rev Drug Discov 1, 131-139 (2002).
25. Blanco, E., Shen, H. & Ferrari, M. Principles of nanoparticle design for overcoming biological barriers to drug delivery. Nat Biotechnol 33, 941-951 (2015).

26. Rezvani, A.R. & Maloney, D.G. Rituximab resistance. Best Pract Res Clin Haematol 24, 203-216 (2011).

27. Abes, R., Gelize, E., Fridman, W.H. & Teillaud, J.L. Long-lasting antitumor protection by anti-CD20 antibody through cellular immune response. Blood 116, 926-934 (2010).

28. Yan, M., et al. A novel intracellular protein delivery platform based on single-protein nanocapsules. Nature Nanotechnology 5, 48-53 (2010).

29. Tian, H., et al. Growth-Factor Nanocapsules That Enable Tunable Controlled Release for Bone Regeneration. ACS Nano 10, 7362-7369 (2016).

30. Liang, S., et al. Phosphorylcholine polymer nanocapsules prolong the circulation time and reduce the immunogenicity of therapeutic proteins. Nano Research 9, 1022-1031 (2016).

31. Zhang, L., et al. Prolonging the plasma circulation of proteins by nanoencapsulation with phosphorylcholine-based polymer. Nano Research 9, 2424-2432 (2016).

32. Young, G., Bowers, R., Hall, B. & Port, M. Six month clinical evaluation of a biomimetic hydrogel contact lens. CLAD 23, 226-236 (1997).

33. Chen, S., Li, L., Zhao, C. & Zheng, J. Surface hydration: Principles and applications toward low-fouling/nonfouling biomaterials. Polymer 51, 5283-5293 (2010).

34. Kato, Y., et al. Acidic extracellular microenvironment and cancer. Cancer Cell Int 13, 89 (2013).

35. Corbet, C. & Feron, O. Tumour acidosis: from the passenger to the driver's seat. Nat Rev Cancer 17, 577-593 (2017).

36. Ito, D., et al. Microglia-specific localisation of a novel calcium binding protein, Iba1. Brain Res Mol Brain Res 57, 1-9 (1998).

37. Rosenberg, G.A., et al. Immunohistochemistry of matrix metalloproteinases in reperfusion injury to rat brain: activation of MMP-9 linked to stromelysin-1 and microglia in cell cultures. Brain Res 893, 104-112 (2001).

38. Widney, D., et al. Levels of murine, but not human, CXCL13 are greatly elevated in NOD-SCID mice bearing the AIDS-associated Burkitt lymphoma cell line, 2F7. PloS One 8, e72414 (2013).

39. Legler, D.F., et al. B cell-attracting chemokine 1, a human CXC chemokine expressed in lymphoid tissues, selectively attracts B lymphocytes via BLR1/CXCR5. J Exp Med 187, 655-660 (1998).

40. Gunn, M.D., et al. A B-cell-homing chemokine made in lymphoid follicles activates Burkitt's lymphoma receptor-1. Nature 391, 799-803 (1998).

41. Charbonneau, B., et al. CXCR5 polymorphisms in non-Hodgkin lymphoma risk and prognosis. Cancer Immunol Immunother 62, 1475-1484 (2013).

42. Hussain, S.K., et al. Serum levels of the chemokine CXCL13, genetic variation in CXCL13 and its receptor CXCR5, and HIV-associated non-hodgkin B-cell lymphoma risk. Cancer Epidemiol Biomarkers Prev 22, 295-307 (2013).

43. Dobner, T., Wolf, I., Emrich, T. & Lipp, M. Differentiation-specific expression of a novel G protein-coupled receptor from Burkitt's lymphoma. Eur J Immunol 22, 2795-2799 (1992).

44. Smith, J.R., et al. Expression of B-cell-attracting chemokine 1 (CXCL13) by malignant lymphocytes and vascular endothelium in primary central nervous system lymphoma. Blood 101, 815-821 (2003).

45. Trentin, L., et al. Homeostatic chemokines drive migration of malignant B cells in patients with non-Hodgkin lymphomas. Blood 104, 502-508 (2004).

46. Shultz, L., et al. Human lymphoid and myeloid cell development in NOD/LtSz-scid IL2R gamma null mice engrafted with mobilized human hemopoietic stem cells. Journal of Immunology 174, 6477-6489 (2005).

47. Chijioke, O., et al. Human natural killer cells prevent infectious mononucleosis features by targeting lytic Epstein-Barr virus infection. Cell Rep 5, 1489-1498 (2013).

48. Melkus, M., et al. Humanized mice mount specific adaptive and innate immune responses to EBV and TSST-1. Nature Med 12, 1316-1322 (2006).

49. Shultz, L.D., Brehm, M.A., Garcia-Martinez, J.V. & Greiner, D.L. Humanized mice for immune system investigation: progress, promise and challenges. Nat Rev Immunol 12, 786-798 (2012).

50. Walsh, N.C., et al. Humanized Mouse Models of Clinical Disease. Annu Rev Pathol 12, 187-215 (2017).

51. Honeycutt, J.B., et al. Macrophages sustain HIV replication in vivo independently of T cells. J Clin Invest 126, 1353-1366 (2016).

52. Gasque, P., Dean, Y.D., McGreal, E.P., VanBeek, J. & Morgan, B.P. Complement components of the innate immune system in health and disease in the CNS. Immunopharmacology 49, 171-186 (2000).

53. Salter, M.W. & Beggs, S. Sublime microglia: expanding roles for the guardians of the CNS. Cell 158, 15-24 (2014).

54. Prinz, M. & Priller, J. Microglia and brain macrophages in the molecular age: from origin to neuropsychiatric disease. Nat Rev Neurosci 15, 300-312 (2014).

55. Stevens, B., et al. The classical complement cascade mediates CNS synapse elimination. Cell 131, 1164-1178 (2007).

56. Lin, N.U., Amiri-Kordestani, L., Palmieri, D., Liewehr, D.J. & Steeg, P.S. CNS metastases in breast cancer: old challenge, new frontiers. Clin Cancer Res 19, 6404-6418 (2013).

57. Zagouri, F., et al. Intrathecal administration of trastuzumab for the treatment of meningeal carcinomatosis in HER2-positive metastatic breast cancer: a systematic review and pooled analysis. Breast Cancer Res Treat 139, 13-22 (2013).

58. Strayer, D.S. & Kohler, H. Immune response to phosphorylcholine II, natural "auto"-anti-receptor antibody in neonatal Balb/c mice. Cell Immunol 25, 294-301 (1976).

59. Su, J., et al. Antibodies of IgM subclass to phosphorylcholine and oxidized LDL are protective factors for atherosclerosis in patients with hypertension. Atherosclerosis 188, 160-166 (2006).

60. Bertolini, F., et al. Endostatin, an antiangiogenic drug, induces tumor stabilization after chemotherapy or anti-CD20 therapy in a NOD/SCID mouse model of human high-grade non-Hodgkin lymphoma. Blood 96, 282-287 (2000).

61. Zhen, A., et al. Stem-cell Based Engineered Immunity Against HIV Infection in the Humanized Mouse Model. J Vis Exp (2016).

62. Zhen, A., et al. HIV-specific immunity derived from chimeric antigen receptor-engineered stem cells. Molecular Therapy 23, 1358-1367 (2015).

63. Ringpis, G., et al. Engineering HIV-1-resistant T-cells from short-hairpin RNA-expressing hematopoietic stem/progenitor cells in humanized BLT mice. PLoS One 7, e53492 (2012).

CONCLUSION

This concludes the description of the preferred embodiment of the present invention. The foregoing description of one or more embodiments of the invention has been presented for the purposes of illustration and description.

**Claims**

1.  A composition of matter comprising:

    an antibody;
    a polymeric network formed from 2-methacryloyloxyethyl phosphorylcholine and configured to form a nanocapsule that encapsulates the antibody;
    a hydrolysable crosslinking moiety comprising a glycerol dimethacrylate and/or a Poly(DL-lactide)-b-Poly(ethylene glycol)-b-Poly(DL-lactide)-diacrylate triblock copolymer coupled to polymers forming the polymeric network; and
    a targeting agent comprising CXCL13 coupled to the polymeric network;
    wherein:
    the nanocapsule is formed *in situ* on the antibody;
    constituents and ratios of the antibody, the polymeric network, the hydrolysable crosslinking moiety and the targeting agent are disposed in a three-dimensional architecture so that:

        the nanocapsule crosses blood brain barriers to deliver nanocapsules in a bloodstream into a central nervous system such that at least 10 fold more antibody that is disposed in the nanocapsule crosses blood brain barriers as compared with the antibody in the absence of the nanocapsule; and
        the hydrolysable crosslinking moiety is cleaved in the central nervous system so as to release the antibody from the nanocapsule.

2.  A method of making the composition according to claim **1,** the method comprising:

    performing an *in situ* polymerization process on monomers that comprise 2-methacryloyloxyethyl phosphorylcholine that form electrostatic interactions with the antibody so as to form a nanocapsule that encapsulates the antibody;
    coupling polymers in the polymeric network formed from 2-methacryloyloxyethyl phosphorylcholine with one or more hydrolysable crosslinking moieties comprising a glycerol dimethacrylate and/or a Poly(DL-lactide)-b-Poly(ethylene glycol)-b-Poly(DL-lactide)-diacrylate triblock copolymer, wherein the hydrolysable crosslinking moieties are selected to be cleaved in the central nervous system so as to release the antibody from the nanocapsule; and
    coupling the polymeric network to one or more targeting agents comprising CXCL13, wherein the targeting agents are selected to bind to targets present in tissues of the central nervous system;
    wherein constituents and ratios of the antibody, the polymeric network formed from 2-methacryloyloxyethyl phosphorylcholine, the hydrolysable crosslinking moiety comprising a glycerol dimethacrylate; and/or a Poly(DL-lactide)-b-Poly(ethylene glycol)-b-Poly(DL-lactide)-diacrylate triblock copolymer and the targeting agent comprising CXCL13 are selected to allow:

the nanocapsule to cross blood brain barriers to deliver protein cargos into a central nervous system; and the hydrolysable crosslinking moiety to be cleaved in the central nervous system so as to release the antibodyfrom the nanocapsule.

3. The method of claim 2, wherein the antibody is selected to bind to cancerous cells.

4. The method of claim 3, wherein the cancerous cells are observed to metastasize to the central nervous system.

5. The composition of claim 1 for use in selectively delivering a therapeutic agent to the central nervous system of an individual wherein said use involves intravenously administering the composition and allowing the composition to cross the blood brain barrier of the individual into the central nervous system.

6. The composition for use according to claim 5 wherein the targeting agent binds to targets on diseased tissue within the central nervous system.

7. The composition for use according to claim 5 wherein the targeting agent is a chemokine.

8. The composition for use according to claim 5 wherein the targeting agent binds to receptors present on neural precursor cells.

9. The composition of claim 1, wherein the antibody is selected to bind to cancerous cells.

10. The composition of claim 9 wherein the cancerous cells are observed to metastasize to the central nervous system.

11. The composition of claim 1, further comprising a plurality of nanocapsules, wherein constituents and ratios of the hydrolysable crosslinking moiety in the plurality of nanocapsules are disposed in a three-dimensional architecture so that the plurality of nanocapsules release the antibody at different rates of time.

**Patentansprüche**

1. Eine Stoffzusammensetzung, die Folgendes beinhaltet:

einen Antikörper;
ein polymeres Netzwerk, das aus 2-Methacryloyloxyethylphosphorylcholin gebildet ist und konfiguriert ist, um eine Nanokapsel zu bilden, die den Antikörper einkapselt;
einen hydrolysierbaren Vernetzungsanteil, der ein Glyceroldimethacrylat und/oder ein Poly(DL-lactid)-b-Poly(ethylenglycol)-b-Poly(DL-lactid)-diacrylat-Triblockcopolymer beinhaltet und mit Polymeren gekoppelt ist, die das polymere Netzwerk bilden; und
ein Zielmittel, das CXCL13 beinhaltet und mit dem polymeren Netzwerk gekoppelt ist; wobei:

die Nanokapsel auf dem Antikörper *in situ* gebildet wird;
Bestandteile und Verhältnisse des Antikörpers, des polymeren Netzwerks, des hydrolysierbaren Vernetzungsanteils und des Zielmittels in einer dreidimensionalen Architektur so angeordnet sind, dass:

die Nanokapsel Blut-Gehirn-Schranken passiert, um Nanokapseln in einer Blutbahn in ein Zentralnervensystem abzugeben, sodass mindestens 10-mal mehr Antikörper, der in der Nanokapsel angeordnet ist, Blut-Gehirn-Schranken passiert als im Vergleich zu dem Antikörper in Abwesenheit der Nanokapsel; und
der hydrolysierbare Vernetzungsanteil in dem Zentralnervensystem aufgespalten wird, sodass der Antikörper aus der Nanokapsel freigesetzt wird.

2. Ein Verfahren zum Herstellen der Zusammensetzung gemäß Anspruch 1, wobei das Verfahren Folgendes beinhaltet:

Durchführen eines In-situ-Polymerisationsprozesses an Monomeren, die 2-Methacryloyloxyethylphosphorylcholin beinhalten, die elektrostatische Wechselwirkungen mit dem Antikörper bilden, sodass eine Nanokapsel gebildet wird, die den Antikörper einkapselt;

Koppeln von Polymeren in dem aus 2-Methacryloyloxyethylphosphorylcholin gebildeten polymeren Netzwerk mit einem oder mehreren hydrolysierbaren Vernetzungsanteilen, die ein Glyceroldimethacrylat und/oder ein Poly(DL-lactid)-b-Poly(ethylenglycol)-b-Poly(DL-lactid)-diacrylat-Triblockcopolymer beinhalten, wobei die hydrolysierbaren Vernetzungsanteile ausgewählt sind, um in dem Zentralnervensystem aufgespalten zu werden, sodass der Antikörper aus der Nanokapsel freigesetzt wird; und

Koppeln des polymeren Netzwerks mit einem oder mehreren Zielmitteln, die CXCL13 beinhalten, wobei die Zielmittel ausgewählt sind, um an Ziele zu binden, die in Geweben des Zentralnervensystems vorhanden sind; wobei Bestandteile und Verhältnisse des Antikörpers, des aus 2-Methacryloyloxyethylphosphorylcholin gebildeten polymeren Netzwerks, des hydrolysierbaren Vernetzungsanteils, der ein Glyceroldimethacrylat und/oder ein Poly(DL-lactid)-b-Poly(ethylenglycol)-b-Poly(DL-lactid)-diacrylat-Triblockcopolymer beinhaltet, und des Zielmittels, das CXCL13 beinhaltet, ausgewählt sind, um Folgendes zu ermöglichen:

das Passieren der Nanokapsel von Blut-Gehirn-Schranken, um Proteincargo in ein Zentralnervensystem abzugeben; und

das Aufspalten des hydrolysierbaren Vernetzungsanteils in dem Zentralnervensystem, sodass der Antikörper aus der Nanokapsel freigesetzt wird.

3. Verfahren gemäß Anspruch 2, wobei der Antikörper ausgewählt ist, um an Krebszellen zu binden.

4. Verfahren gemäß Anspruch 3, wobei das Metastasieren der Krebszellen in das Zentralnervensystem beobachtet wird.

5. Zusammensetzung gemäß Anspruch 1 zur Verwendung bei der selektiven Abgabe eines Therapeutikums an das Zentralnervensystem eines Individuums, wobei die Verwendung die intravenöse Verabreichung der Zusammensetzung und das Ermöglichen des Passierens der Zusammensetzung durch die Blut-Gehirn-Schranke des Individuums in das Zentralnervensystem involviert.

6. Zusammensetzung zur Verwendung gemäß Anspruch 5, wobei das Zielmittel an Ziele auf erkranktem Gewebe innerhalb des Zentralnervensystems bindet.

7. Zusammensetzung zur Verwendung gemäß Anspruch 5, wobei das Zielmittel ein Chemokin ist.

8. Zusammensetzung zur Verwendung gemäß Anspruch 5, wobei das Zielmittel an Rezeptoren bindet, die auf neuralen Vorläuferzellen vorhanden sind.

9. Zusammensetzung gemäß Anspruch 1, wobei der Antikörper ausgewählt ist, um an Krebszellen zu binden.

10. Zusammensetzung gemäß Anspruch 9, wobei das Metastasieren der Krebszellen in das Zentralnervensystem beobachtet wird.

11. Zusammensetzung gemäß Anspruch 1, die ferner eine Vielzahl von Nanokapseln beinhaltet, wobei Bestandteile und Verhältnisse des hydrolysierbaren Vernetzungsanteils in der Vielzahl von Nanokapseln in einer dreidimensionalen Architektur angeordnet sind, sodass die Vielzahl von Nanokapseln den Antikörper mit unterschiedlichen zeitlichen Raten freisetzen.

## Revendications

1. Une composition de matière comprenant :

un anticorps ;

un réseau polymère formé à partir de 2-méthacryloyloxyéthyl phosphorylcholine et configuré pour former une nanocapsule qui encapsule l'anticorps ;

un fragment de réticulation hydrolysable comprenant un diméthacrylate de glycérol et/ou un copolymère tribloc Poly(DL-lactide)-b-Poly(éthylène glycol)-b-Poly(DL-lactide)-diacrylate couplé à des polymères formant le réseau polymère ; et
un agent de ciblage comprenant CXCL13 couplé au réseau polymère ; où :

la nanocapsule est formée *in situ* sur l'anticorps ;
des constituants et rapports de l'anticorps, du réseau polymère, du fragment de réticulation hydrolysable et de l'agent de ciblage sont disposés en une architecture en trois dimensions de sorte que :

la nanocapsule traverse des barrières hématoencéphaliques afin de délivrer des nanocapsules dans une circulation sanguine jusque dans un système nerveux central de telle sorte qu'au moins 10 fois plus d'un anticorps qui est disposé dans la nanocapsule traverse les barrières hématoencéphaliques par comparaison avec l'anticorps en l'absence de la nanocapsule ;
le fragment de réticulation hydrolysable est clivé dans le système nerveux central de façon à libérer l'anticorps de la nanocapsule.

2. Un procédé de fabrication de la composition selon la revendication 1, le procédé comprenant :

le fait d'effectuer un processus de polymérisation *in situ* sur des monomères qui comprennent de la 2-méthacryloyloxyéthyl phosphorylcholine qui forment des interactions électrostatiques avec l'anticorps de façon à former une nanocapsule qui encapsule l'anticorps ;
le fait de coupler des polymères dans le réseau polymère formé à partir de 2-méthacryloxyloxyéthyl phosphorylcholine avec un ou plusieurs fragments de réticulation hydrolysables comprenant un diméthacrylate de glycérol et/ou un copolymère tribloc Poly(DL-lactide)-b-Poly(éthylène glycol)-b-Poly(DL-lactide)-diacrylate, où les fragments de réticulation hydrolysables sont sélectionnés afin d'être clivés dans le système nerveux central de façon à libérer l'anticorps de la nanocapsule ; et
le fait de coupler le réseau polymère à un ou plusieurs agents de ciblage comprenant CXCL13, où les agents de ciblage sont sélectionnés afin de se lier à des cibles présentes dans des tissus du système nerveux central ; où des constituants et rapports de l'anticorps, du réseau polymère formé à partir de 2-méthacryloxyloxyéthyl phosphorylcholine, du fragment de réticulation hydrolysable comprenant un diméthacrylate de glycérol et/ou un copolymère tribloc Poly(DL-lactide)-b-Poly(éthylène glycol)-b-Poly(DL-lactide)-diacrylate et de l'agent de ciblage comprenant CXCL13 sont sélectionnés afin de permettre :

à la nanocapsule de traverser les barrières hématoencéphaliques afin de délivrer des cargaisons protéiques jusque dans un système nerveux central ; et
au fragment de réticulation hydrolysable d'être clivé dans le système nerveux central de façon à libérer l'anticorps de la nanocapsule.

3. Le procédé de la revendication 2, où l'anticorps est sélectionné afin de se lier à des cellules cancéreuses.

4. Le procédé de la revendication 3, où les cellules cancéreuses sont observées comme métastasant dans le système nerveux central.

5. La composition de la revendication 1 pour une utilisation dans le fait de délivrer sélectivement un agent thérapeutique au système nerveux central d'un individu, où ladite utilisation implique le fait d'administrer par voie intraveineuse la composition et le fait de permettre à la composition de traverser la barrière hématoencéphalique de l'individu jusque dans le système nerveux central.

6. La composition pour une utilisation selon la revendication 5 où l'agent de ciblage se lie à des cibles sur le tissu malade au sein du système nerveux central.

7. La composition pour une utilisation selon la revendication 5 où l'agent de ciblage est une chimiokine.

8. La composition pour une utilisation selon la revendication 5 où l'agent de ciblage se lie à des récepteurs présents sur des cellules précurseurs neurales.

9. La composition de la revendication 1, où l'anticorps est sélectionné afin de se lier à des cellules cancéreuses.

10. La composition de la revendication 9 où les cellules cancéreuses sont observées comme métastasant dans le système nerveux central.

11. La composition de la revendication 1, comprenant en outre une pluralité de nanocapsules, où des constituants et rapports du fragment de réticulation hydrolysable dans la pluralité de nanocapsules sont disposés en une architecture en trois dimensions de sorte que les nanocapsules de la pluralité de nanocapsules libèrent l'anticorps à des rythmes différents.

MPC

PLA-PEG-PLA

GDMA

FIG. 1A

FIG. 1B

FIG. 1C

FIG. 1D

FIG. 1E

## Fig.2

FIG. 3A

FIG. 3B

FIG. 3C

FIG. 3D

FIG. 3E

FIG. 3F

Fig.4

Fig.5

Fig.6

EP 3 927 366 B1

Fig. 7

Fig. 8

Fig. 9

Fig. 10

Fig. 11

Fig. 12

Fig. 13

Fig. 14

A

B

EP 3 927 366 B1

Fig. 15

Fig. 16

Fig. 17

Fig. 18

Bright-field     2F7-BR44     Overlay

Fig. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3424498 A

**Non-patent literature cited in the description**

- **QIN et al.** Enhanced Delivery of Rituximab Into Brain and Lymph Nodes Using Timed-Release Nanocapsules in Non-Human Primates. *Nature Biomedical Engineering,* 2019, 706-716
- **CHAMBERS, A.F. ; GROOM, A.C. ; MACDONALD, I.C.** Dissemination and growth of cancer cells in metastatic sites. *Nat Rev Cancer,* 2002, vol. 2, 563-572
- **ARAGON-CHING, J.B. ; ZUJEWSKI, J.A.** CNS metastasis: an old problem in a new guise. *Clin Cancer Res,* 2007, vol. 13, 1644-1647
- **TOSONI, A. ; ERMANI, M. ; & ; BRANDES, A.A.** The pathogenesis and treatment of brain metastases: a comprehensive review. *Crit Rev Oncol Hematol,* 2004, vol. 52, 199-215
- **RUBENSTEIN, J.L. et al.** Rituximab therapy for CNS lymphomas: targeting the leptomeningeal compartment. *Blood,* 2003, vol. 101, 466-468
- **ZHANG, Y. ; PARDRIDGE, W.M.** Mediated efflux of IgG molecules from brain to blood across the blood-brain barrier. *J Neuroimmunol,* 2001, vol. 114, 168-172
- **CZYZEWSKI, K. et al.** Intrathecal therapy with rituximab in central nervous system involvement of post-transplant lymphoproliferative disorder. *Leuk Lymphoma,* 2013, vol. 54, 503-506
- **LU, N.T. et al.** Intrathecal trastuzumab: immunotherapy improves the prognosis of leptomeningeal metastases in HER-2+ breast cancer patient. *J Immunother Cancer,* 2015, vol. 3, 41
- **COOPER, P.R. et al.** Efflux of monoclonal antibodies from rat brain by neonatal Fc receptor, FcRn. *Brain Res,* 2013, vol. 1534, 13-21
- **RUBENSTEIN, J.L. et al.** Multicenter phase 1 trial of intraventricular immunochemotherapy in recurrent CNS lymphoma. *Blood,* 2013, vol. 121, 745-751
- **RUBENSTEIN, J.L. et al.** Phase I study of intraventricular administration of rituximab in patients with recurrent CNS and intraocular lymphoma. *J Clin Oncol,* 2007, vol. 25, 1350-1356
- **BOUSQUET, G. et al.** Intrathecal Trastuzumab Halts Progression of CNS Metastases in Breast Cancer. *J Clin Oncol,* 2016, vol. 34, e151-155
- **CHEN, Y. ; LIU, L.** Modern methods for delivery of drugs across the blood-brain barrier. *Adv Drug Deliv Rev,* 2012, vol. 64, 640-665
- **SCHROEDER, A. et al.** Treating metastatic cancer with nanotechnology. *Nat Rev Cancer,* 2011, vol. 12, 39-50
- **LU, C.T. et al.** Current approaches to enhance CNS delivery of drugs across the brain barriers. *Int J Nanomedicine,* 2014, vol. 9, 2241-2257
- **FU, H. ; MCCARTY, D.M.** Crossing the blood-brain-barrier with viral vectors. *Curr Opin Virol,* 2016, vol. 21, 87-92
- **SPENCER, B.J. ; VERMA, I.M.** Targeted delivery of proteins across the blood-brain barrier. *Proc Natl Acad Sci USA,* 2007, vol. 104, 7594-7599
- **BATTAGLIA, L. ; GALLARATE, M.** Lipid nanoparticles: state of the art, new preparation methods and challenges in drug delivery. *Expert Opin Drug Deliv,* 2012, vol. 9, 497-508
- **HELM, F. ; FRICKER, G.** Liposomal conjugates for drug delivery to the central nervous system. *Pharmaceutics,* 2015, vol. 7, 27-42
- **KREUTER, J.** Drug delivery to the central nervous system by polymeric nanoparticles: what do we know?. *Adv Drug Deliv Rev,* 2014, vol. 71, 2-14
- **MISHRA, B. ; PATEL, B.B. ; TIWARI, S.** Colloidal nanocarriers: a review on formulation technology, types and applications toward targeted drug delivery. *Nanomedicine,* 2010, vol. 6, 9-24
- **PAPASANI, M.R. ; WANG, G. ; HILL, R.A.** Gold nanoparticles: the importance of physiological principles to devise strategies for targeted drug delivery. *Nanomedicine,* 2012, vol. 8, 804-814
- **CLARK, A.J. ; DAVIS, M.E.** Increased brain uptake of targeted nanoparticles by adding an acid-cleavable linkage between transferrin and the nanoparticle core. *Proc Natl Acad Sci U S A,* 2015, vol. 112, 12486-12491
- **BHARALI, D.J. et al.** Organically modified silica nanoparticles: a nonviral vector for in vivo gene delivery and expression in the brain. *Proc Natl Acαd Sci U S A,* 2005, vol. 102, 11539-11544

- **PARDRIDGE, W.M.** Drug and gene targeting to the brain with molecular Trojan horses. *Nat Rev Drug Discov,* 2002, vol. 1, 131-139
- **BLANCO, E. ; SHEN, H. ; FERRARI, M.** Principles of nanoparticle design for overcoming biological barriers to drug delivery. *Nat Biotechnol,* 2015, vol. 33, 941-951
- **REZVANI, A.R. ; MALONEY, D.G.** Rituximab resistance. *Best Pract Res Clin Haematol,* 2011, vol. 24, 203-216
- **ABES, R. ; GELIZE, E. ; FRIDMAN, W.H. ; TEILLAUD, J.L.** Long-lasting antitumor protection by anti-CD20 antibody through cellular immune response. *Blood,* 2010, vol. 116, 926-934
- **YAN, M. et al.** A novel intracellular protein delivery platform based on single-protein nanocapsules. *Nature Nanotechnology,* 2010, vol. 5, 48-53
- **TIAN, H. et al.** Growth-Factor Nanocapsules That Enable Tunable Controlled Release for Bone Regeneration. *ACS Nano,* 2016, vol. 10, 7362-7369
- **LIANG, S. et al.** Phosphorylcholine polymer nanocapsules prolong the circulation time and reduce the immunogenicity of therapeutic proteins. *Nano Research,* 2016, vol. 9, 1022-1031
- **ZHANG, L. et al.** Prolonging the plasma circulation of proteins by nanoencapsulation with phosphorylcholine-based polymer. *Nano Research,* 2016, vol. 9, 2424-2432
- **YOUNG, G. ; BOWERS, R. ; HALL, B. ; PORT, M.** Six month clinical evaluation of a biomimetic hydrogel contact lens. *CLAD,* 1997, vol. 23, 226-236
- **CHEN, S. ; LI, L. ; ZHAO, C ; ZHENG, J.** Surface hydration: Principles and applications toward low-fouling/nonfouling biomaterials. *Polymer,* 2010, vol. 51, 5283-5293
- **KATO, Y. et al.** Acidic extracellular microenvironment and cancer. *Cancer Cell Int,* 2013, vol. 13 (89
- **CORBET, C. ; FERON, O.** Tumour acidosis: from the passenger to the driver's seat. *Nat Rev Cancer,* 2017, vol. 17, 577-593
- **ITO, D. et al.** Microglia-specific localisation of a novel calcium binding protein, Iba1. *Brain Res Mol Brain Res,* 1998, vol. 57, 1-9
- **ROSENBERG, G.A. et al.** Immunohistochemistry of matrix metalloproteinases in reperfusion injury to rat brain: activation of MMP-9 linked to stromelysin-1 and microglia in cell cultures. *Brain Res,* 2001, vol. 893, 104-112
- **WIDNEY, D. et al.** Levels of murine, but not human, CXCL13 are greatly elevated in NOD-SCID mice bearing the AIDS-associated Burkitt lymphoma cell line, 2F7. *PloS One,* 2013, vol. 8, e72414
- **LEGLER, D.F. et al.** B cell-attracting chemokine 1, a human CXC chemokine expressed in lymphoid tissues, selectively attracts B lymphocytes via BLR1/CXCR5. *J Exp Med,* 1998, vol. 187, 655-660
- **GUNN, M.D. et al.** A B-cell-homing chemokine made in lymphoid follicles activates Burkitt's lymphoma receptor-1. *Nature,* 1998, vol. 391, 799-803
- **CHARBONNEAU, B. et al.** CXCR5 polymorphisms in non-Hodgkin lymphoma risk and prognosis. *Cancer Immunol Immunother,* 2013, vol. 62, 1475-1484
- **HUSSAIN, S.K. et al.** Serum levels of the chemokine CXCL13, genetic variation in CXCL13 and its receptor CXCR5, and HIV-associated non-hodgkin B-cell lymphoma risk. *Cancer Epidemiol Biomarkers Prev,* 2013, vol. 22, 295-307
- **DOBNER, T. ; WOLF, I. ; EMRICH, T. ; LIPP, M.** Differentiation-specific expression of a novel G protein-coupled receptor from Burkitt's lymphoma. *Eur J Immunol,* 1992, vol. 22, 2795-2799
- **SMITH, J.R. et al.** Expression of B-cell-attracting chemokine 1 (CXCL13) by malignant lymphocytes and vascular endothelium in primary central nervous system lymphoma. *Blood,* 2003, vol. 101, 815-821
- **TRENTIN, L. et al.** Homeostatic chemokines drive migration of malignant B cells in patients with non-Hodgkin lymphomas. *Blood,* 2004, vol. 104, 502-508
- **SHULTZ, L. et al.** Human lymphoid and myeloid cell development in NOD/LtSz-scid IL2R gamma null mice engrafted with mobilized human hemopoietic stem cells. *Journal of Immunology,* 2005, vol. 174, 6477-6489
- **CHIJIOKE, O. et al.** Human natural killer cells prevent infectious mononucleosis features by targeting lytic Epstein-Barr virus infection. *Cell Rep,* 2013, vol. 5, 1489-1498
- **MELKUS, M. et al.** Humanized mice mount specific adaptive and innate immune responses to EBV and TSST-1. *Nature Med,* 2006, vol. 12, 1316-1322
- **SHULTZ, L.D. ; BREHM, M.A. ; GARCIA-MARTINEZ, J.V. ; GREINER, D.L.** Humanized mice for immune system investigation: progress, promise and challenges. *Nat Rev Immunol,* 2012, vol. 12, 786-798
- **WALSH, N.C. et al.** Humanized Mouse Models of Clinical Disease. *Annu Rev Pathol,* 2017, vol. 12, 187-215
- **HONEYCUTT, J.B. et al.** Macrophages sustain HIV replication in vivo independently of T cells. *J Clin Invest,* 2016, vol. 126, 1353-1366
- **GASQUE, P. ; DEAN, Y.D. ; MCGREAL, E.P. ; VANBEEK, J. ; MORGAN, B.P.** Complement components of the innate immune system in health and disease in the CNS. *Immunopharmacology,* 2000, vol. 49, 171-186
- **SALTER, M.W. ; BEGGS, S.** Sublime microglia: expanding roles for the guardians of the CNS. *Cell,* 2014, vol. 158, 15-24

- **PRINZ, M. ; PRILLER, J.** Microglia and brain macrophages in the molecular age: from origin to neuropsychiatric disease. *Nat Rev Neurosci,* 2014, vol. 15, 300-312
- **STEVENS, B. et al.** The classical complement cascade mediates CNS synapse elimination. *Cell,* 2007, vol. 131, 1164-1178
- **LIN, N.U. ; AMIRI-KORDESTANI, L. ; PALMIERI, D. ; LIEWEHR, D.J. ; STEEG, P.S.** CNS metastases in breast cancer: old challenge, new frontiers. *Clin Cancer Res,* 2013, vol. 19, 6404-6418
- **ZAGOURI, F. et al.** Intrathecal administration of trastuzumab for the treatment of meningeal carcinomatosis in HER2-positive metastatic breast cancer: a systematic review and pooled analysis. *Breast Cancer Res Treat,* 2013, vol. 139, 13-22
- **STRAYER, D.S. ; KOHLER, H.** Immune response to phosphorylcholine II, natural ''auto''-anti-receptor antibody in neonatal Balb/c mice. *Cell Immunol,* 1976, vol. 25, 294-301
- **SU, J. et al.** Antibodies of IgM subclass to phosphorylcholine and oxidized LDL are protective factors for atherosclerosis in patients with hypertension. *Atherosclerosis,* 2006, vol. 188, 160-166
- **BERTOLINI, F. et al.** Endostatin, an antiangiogenic drug, induces tumor stabilization after chemotherapy or anti-CD20 therapy in a NOD/SCID mouse model of human high-grade non-Hodgkin lymphoma. *Blood,* 2000, vol. 96, 282-287
- **ZHEN, A. et al.** Stem-cell Based Engineered Immunity Against HIV Infection in the Humanized Mouse Model. *J Vis Exp,* 2016
- **ZHEN, A. et al.** HIV-specific immunity derived from chimeric antigen receptor-engineered stem cells. *Molecular Therapy,* 2015, vol. 23, 1358-1367
- **RINGPIS, G. et al.** Engineering HIV-1-resistant T-cells from short-hairpin RNA-expressing hematopoietic stem/progenitor cells in humanized BLT mice. *PLoS One,* 2012, vol. 7, e53492